# EUROPEAN PATENT SPECIFICATION

(11) **EP 2 576 772 B1**
(45) Date of publication and mention of the grant of the patent: **12.12.2018**
(21) Application number: 11716853.4
(22) Date of filing: 04.04.2011
(51) Int. Cl.: A61K 35/17, A61K 39/00, C12N 5/0781, A61K 35/12

(54) **ANTIGEN-PRESENTING MODIFIED NAÏVE B CELLS FOR IMMUNE SUPPRESSION AND A METHOD FOR PRODUCING SAID MODIFIED CELLS**
INAKTIVE B-ZELLEN MIT ANTIGENEN ZUR IMMUNUNTERDRÜCKUNG UND HERSTELLUNGSVERFAHREN FÜR DIE ZELLEN
LYMPHOCYTES B INACTIVÉS PRÉSENTANT UN ANTIGÈNE POUR LA SUPPRESSION IMMUNITAIRE ET PROCÉDÉ DE PRODUCTION DE CES CELLULES

(30) Priority: 26.05.2010 EP 10075225
(43) Date of publication of application: 10.04.2013
(73) Proprietor: Deutsches Rheuma-Forschungszentrum Berlin, 10117 Berlin (DE)
(72) Inventor: FILLATREAU, Simon, 10117 Berlin (DE); CALDERON-GOMEZ, Elisabeth, 10117 Berlin (DE)
(74) Representative: Hertin und Partner Rechts- und Patentanwälte PartG mbB
(86) International application number: PCT/EP2011/055208
(87) International publication number: WO 2011/147622

(56) References cited:
- WO-A2-2009/047270
- WARNCKE M ET AL: "Efficient in vitro transduction of naive murine B cells with lentiviral vectors", BIOCHEMICAL AND BIOPHYSICAL RESEARCH COMMUNICATIONS, ACADEMIC PRESS INC. ORLANDO, FL, US LNKD- DOI:10.1016/J.BBRC.2004.04.057, vol. 318, no. 3, 4 June 2004 (2004-06-04), pages 673-679, XP004508486, ISSN: 0006-291X
- FRECHA CECILIA ET AL: "Efficient and stable transduction of resting B lymphocytes and primary chronic lymphocyte leukemia cells using measles virus gp displaying lentiviral vectors", BLOOD, vol. 114, no. 15, October 2009 (2009-10), pages 3173-3180, XP009140779, ISSN: 0006-4971
- LEI TIE CHI ET AL: "Induction of tolerance to factor VIII inhibitors by gene therapy with immunodominant A2 and C2 domains presented by B cells as Ig fusion proteins", BLOOD, AMERICAN SOCIETY OF HEMATOLOGY, US LNKD- DOI:10.1182/BLOOD-2004-11-4274, vol. 105, no. 12, 15 June 2005 (2005-06-15), pages 4865-4870, XP009138824, ISSN: 0006-4971 [retrieved on 2005-03-15]
- SU YAN ET AL: "B cells induce tolerance by presenting endogenous peptide-IgG on MHC class II molecules via an IFN-gamma-inducible lysosomal thiol reductase-dependent pathway", JOURNAL OF IMMUNOLOGY, AMERICAN ASSOCIATION OF IMMUNOLOGISTS, US, vol. 181, no. 2, 15 July 2008 (2008-07-15) , pages 1153-1160, XP009138817, ISSN: 0022-1767
- SOUKHAREVA N ET AL: "Treatment of diabetes in NOD mice by gene transfer of Ig-fusion proteins into B cells: Role of T regulatory cells", CELLULAR IMMUNOLOGY, ACADEMIC PRESS, SAN DIEGO, CA, US LNKD- DOI:10.1016/J.CELLIMM.2006.06.004, vol. 240, no. 1, 1 March 2006 (2006-03-01) , pages 41-46, XP024943242, ISSN: 0008-8749 [retrieved on 2006-03-01]
- MELO MARCO E F ET AL: "Gene transfer of Ig-fusion proteins into B cells prevents and treats autoimmune diseases", JOURNAL OF IMMUNOLOGY, AMERICAN ASSOCIATION OF IMMUNOLOGISTS, US, vol. 168, no. 9, 1 May 2002 (2002-05-01), pages 4788-4795, XP002535132, ISSN: 0022-1767
- SKUPSKY JONATHAN ET AL: "Tolerance induction by gene transfer to lymphocytes", CURRENT GENE THERAPY, BENTHAM SCIENCE PUBLISHERS LTD, NL, vol. 7, no. 5, 1 October 2007 (2007-10-01) , pages 369-380, XP009138829, ISSN: 1566-5232
- JUNG ET AL: "Efficient gene transfer into normal human B lymphocytes with the chimeric adenoviral vector AD5/F35", JOURNAL OF IMMUNOLOGICAL METHODS, ELSEVIER SCIENCE PUBLISHERS B.V.,AMSTERDAM, NL, vol. 304, no. 1-2, 1 September 2005 (2005-09-01), pages 78-87, XP005065139, ISSN: 0022-1759

## Description

The invention relates to modified antigen-presenting naive B cells, characterised in that at least one antigen and/or epitopes thereof are presented on the cell surface, whereby the presented antigen(s) is encoded by and expressed from a lentiviral vector within said modified naive B cell, wherein said cells exhibit immune supressing properties against an antigen-specific immune reaction directed against the antigen encoded by said vector. Subject matter of the present invention also includes a method for producing modified non-activated antigen-presenting B cells by isolation of non-activated B cells, preferably by isolation of CD19+ cells, followed by modification of the isolated B cells with an exogenous nucleic acid, preferably by transduction of a lentiviral vector, which encodes the antigen to be presented.

The invention also encompasses the use a lentiviral vector pDBR or a derivative thereof, encoding an antigen of interest for the production of antigen-presenting resting B cells and the use of modified B cells according to the present invention for the suppression of immune reactions, preferably for the suppression of antigen-specific immune reactions directed against the antigen encoded by the exogenous nucleic acid. Also included in the invention are engineered naive regulatory B cells modified to express an antigen, which additionally express immunomodulatory molecules such as suppressive cytokines (for example IL-10, IL-35, TGF-Beta) or other immune modulators, either with or without modulation of antigen-presenting machinery.

### BACKGROUND OF THE INVENTION

Recent studies have identified a new role for B cells as potent immunosuppressive cells *in vivo.* The regulatory functions of B cells are of therapeutic interest in the inhibition of unwanted immune responses, such pathogenic autoimmune responses, or anti-self or anti-graft reactions (or abnormal immune reactions to foreign antigens as in allergy, or immune responses against therapeutic proteins). In support of this notion, B cells can protect recipient mice from several experimental autoimmune diseases upon adoptive transfer. Furthermore, there is evidence that human B cells display similar regulatory activities.

B cells must be activated via Toll-like-receptors (TLR), B cell receptor for antigen (BCR), and CD40 to suppress ongoing autoimmune diseases in mice (PMID: 12244307, 18354200, 8-digit numbers in brackets refer to Pubmed identification numbers (PMID: PubMed - indexed for MEDLINE)). These activation pathways can therefore be used to induce B cells with the suppressive functions necessary to inhibit immunopathology upon administration into patients (17979683). In keeping with this, B cells activated with selected TLR agonists effectively suppressed various autoimmune diseases upon adoptive transfer in recipient mice (18354200, 17979683). However, only some activation protocols conferred regulatory properties to B cells, and the protective mechanisms were then not identified. It is presently not possible to predict whether an *ex vivo* activated B cell will mediate suppressive function upon adoptive transfer in recipient animals. Consequently, when using the methods of the prior art the utilization of activated B cells is unsafe for clinical application due to a potential aggravation of autoimmune symptoms in treated patients.

In contrast to activated B cells, resting B cells express lower levels of stimulatory molecules for T cells such as major histocompatibility complex (MHC)-II, and co-stimulatory receptors CD80 and CD86. They are therefore less immunogenic than activated B cells and should provide a safer substrate for adoptive cellular therapies (2562848, 2973060). Notably, they can block immunity by inducing tolerance in resting T cells, even though they are less effective than activated B cells at inhibiting ongoing immune reactions (1730913, 1740660, 18832734, 1730913, 9036963).

Thus, the therapeutic B cells of the present invention combine the weak immunogenicity of resting B cells with the effective suppressive functions of activated B cells. In order to construct such "resting regulatory B cells" a novel gene therapy protocol has been established and is an aspect of the present invention. This approach allows the genetic re-programming of resting B cells while keeping them in a quiescent state. This enables the production of resting B cells that present antigen to reactive T cells and secrete the immune modulating cytokine interleukin (IL)-10, which are two key features of activated immunosuppressive B cells (12244307).

The present invention encompasses methods whereby re-programmed resting B cells suppress (via antigen-presentation) unwanted immune reactions mediated by CD4 T cells, CD8 T cells, and B cells upon adoptive transfer in recipients. They also protect mouse model recipients from chronic disease (paralysis) in experimental autoimmune encephalomyelitis (EAE), a model of multiple sclerosis in humans (MS). The present invention encompasses resting B cells that can be genetically re-programmed in less than 6 hours for the manipulation of immunity. This approach is of interest in the fields of autoimmunity, allergy, transplantation, treatment with therapeutic protein and gene therapy, to avoid degradation of self or therapeutic tissues by the immune system.

The prior art discloses several approaches to induce antigen-specific tolerance in several autoimmune diseases, such as soluble antigen based approaches, which may have a risk of provoking anaphylactic reactions, and cell-based approaches (cells coated with peptides). The prior art approaches are based on peptides, and require knowledge of relevant epitopes within a specific protein. The present invention exhibits the advantage however, that it can be based on autologous cell transfer that does not require any knowledge of the relevant epitopes. In the context of the present invention it is not important to know the relevant epitopes within the protein because we can express the whole protein in the modified B cells. A further advantage is the reduced time required (it takes less than a day to prepare the modified B cells).

Different B cells are known in the art, which have been transduced with a lentiviral vector tool to govern efficient and stable gene delivery into quiescent B lymphocytes (Frecha C, et al., Blood 2009, 114(15):3173-80). The naive and memory phenotypes of transduced resting B cells were maintained. However, no mention is made of antigen presentation or of functional effects on the immune system.

Furthermore, Warncke M., et al. (Biochemical and Biophysical Research Communications 2004, 318(3):673-9) describes the transduction of naive B cells with a lentiviral vector expressing either GFP or CTLA4Ig. Expression of GFP and CD86 were analysed via FACS in order to determine transduction efficiency and B cell activation, respectively. Transduction experiments were carried out with and without LPS. The results indicate that B cells can be efficiently transduced by lentiviral VSV-G-pseudotyped vectors without B cell activation. Presentation of either of the transduced antigens (GFP or CTLA for Ig) is not described. There was no demonstration that the B cells could affect immune responses to the expressed antigens.

There is a distinct absence in the prior art of antigen-presentation of peptides or epitopes encoded by transduced vectors, in cases where the naive phenotype of the B cells is maintained. Although lentiviral transduction methods are described, which do not rely on LPS-mediated B cell activation, there is no evidence that the transduced antigens are subsequently presented on the cell surface.

Furthermore, there is no prior art that describes the induction of immune tolerance using transduced B cells that retain a naive state. There is therefore also a lack of functional evidence that the cells described in the prior art (transduced naive B cells) present the antigens encoded by the transduced vectors. There is no evidence that the transduced antigens as described in Warncke et al or Frecha et al are processed and presented on the cell surface. Until now, only activated B cells (using LPS-mediated activation) were capable of antigen-presentation and subsequent induction of immune tolerance. The conceptual framework demonstrated in the prior art until now is that antigen-presentation is directly related to B cell activation. Therefore the prior art discloses either B cells that are antigen-presenting and activated, typically using LPS-mediated activation, or B cells which are naive and do not present antigen.

The present invention demonstrates the surprising advantage that not only can resting B cells be transduced, but they can also present the antigen or corresponding epitopes thereof encoded by the transduced nucleic acid molecule, while still maintaining the inactive or resting state. This effect provides the unexpected advantage that the B cells of the present invention can therefore be administered to patients in order to suppress immune reactions that are directed specifically against the antigen encoded by the transduced nucleic acid.

Suitable lentiviral gene transfer vectors are also known in the art, for example vectors disclosed in WO 2009/019612, which are suitable for administration in a host and for medicinal application, especially regarding vaccination against Human Immunodeficiency Virus (HIV). Such vectors can also be applied in gene therapy approaches to induce specific immunity by expressing an antigen to which an immune response is stimulated. Such vectors have however not been applied for the specific task of transducing resting B cells, especially not for transducing resting B cells in the specific context of the present invention, for example for the production of modified B cells capable of immune system modulation. The present invention represents a novel application of such vectors in light of the prior art in that preferably a lentiviral vector can be applied to drive antigen expression in resting B cells, which subsequently leads to antigen presentation. The vector of the present invention is therefore particularly suited for the function as disclosed herein, as it is capable of expressing the appropriate levels of antigen for antigen-presentation by the transduced B cell and therefore generating the subsequent antigen-specific immune suppression, while maintaining B cells in a resting state.

### SUMMARY OF THE INVENTION

In light of the prior art the technical problem underlying the present invention is to provide a, preferably antigen-specific, immune suppressant using modified antigen-presenting naive B cells.

This problem is solved by the features of the independent claims. Preferred embodiments of the present invention are provided by the dependent claims.

The invention therefore relates to modified naive B cells, characterised in that
- at least one antigen and/or epitopes thereof are presented on the cell surface, whereby
- the presented antigen(s) is encoded by and expressed from a lentiviral vector within said modified naive B cell,
wherein said cells exhibit immune supressing properties against an antigen-specific immune reaction directed against the antigen encoded by said vector.

According to the present invention, naive B cells can be genetically engineered without inducing their activation, therefore preserving a resting or non-activated phenotype after modification. The present invention applies genetic modification to engineer "resting regulatory B cells", which are capable of suppressing unwanted antigen-specific T and B-cell immune responses, and production of specific antibodies *in vivo.*

It is an object of the invention to provide resting B cells that are genetically engineered to present transduced antigen and exhibit immune tolerance inducing properties. The naive B cells present specific antigens in the absence of any immune stimulation, wherein the antigens are preferably associated with modulation of an immune response. The antigens according to the invention can not be designated as marker molecules for example for testing transduction efficiency. Importantly, the modified B cells do not up-regulate molecules with key stimulatory roles upon transduction. The modified B cells of the present invention are characterised in that the B cells remain naive after transformation or modification with an endogenous nucleic acid encoding the antigen to be presented.

The modified B cells of the present invention are characterised in that the exogenous nucleic acid used to transform the B cells is a lentiviral vector, more preferably an HIV-based lentiviral vector.

In a preferred embodiment the modified B cells of the present invention are characterised in that they express similar levels of MHC-II, CD40, CD44, CD69, CD80, and/or CD86 as endogenous naive B cells, and preferably do not produce IL-6. The manipulation of the B cells per se does not alter the expression of these molecules, but their expression can be especially deliberately changed to improve the suppressive function of the engineered naive B cells.

In a further embodiment the modified B cells of the present invention are characterised in that the antigen of interest and/or epitopes thereof are presented via an antigen-presenting molecule on the surface of the modified B cells, such as MHC-II. In another embodiment the modified B cells are capable of presenting the antigen of interest or epitopes thereof to MHC-II-restricted CD4+ T cells.

The invention therefore encompasses the use of a lentiviral vector which has been modified so that a DNA molecule that encodes an antigen of interest has been inserted into the vector. This antigen is subsequently expressed from the vector (post-transduction), so that the antigen is presented on the surface of the transduced naive B cells according to the present invention. The invention therefore relates to B cells that induce immune tolerance, or repression of an immune response, towards the antigen of interest, which is encoded by a nucleic acid sequence that has been inserted into the vector as described herein. The modification of the vector with the antigen-encoding nucleic acid molecule can occur via methods known to one skilled in the art, such as recombinant DNA technology, involving for example standard cloning techniques such as restriction digestion and ligation or recombination-based approaches used to insert the antigen-coding DNA. References in this document to vectors, which encode and express an antigen to be presented, relate to vectors that have been modified, so that DNA sequence, which encodes an antigen of interest, has been inserted.

In one embodiment of the invention the modified B cells secrete immunosuppressive molecules, such as IL-10, at levels higher than endogenous non-activated B cells.

Another aspect of the invention is a method for producing modified antigen-presenting naive B cells, characterised by providing isolated naive B cells, preferably by isolation of CD19+ cells, followed by transformation of the isolated B cells by transduction of a lentiviral vector, which encodes the antigen to be presented.

Disclosed herein is the lentiviral vector pDBR, especially according to SEQ ID NO 1 or a derivative thereof.

A further aspect of the invention is the use of pDBR according to SEQ ID NO 1 or a derivative thereof, which encodes an antigen to be presented, for the transformation of non-activated B cells and the production of modified antigen-presenting naive B cells according to the present invention.

Disclosed herein is a nucleic acid sequence, wherein the sequence is selected from the group consisting of:
a) a nucleic acid molecule according to SEQ ID NO 1 or complementary nucleotide sequences thereof,
b) a nucleic acid molecule hybridizing with a nucleotide sequence according to a) under stringent conditions,
c) a nucleic acid molecule comprising a nucleotide sequence having sufficient homology to be functionally analogous to a nucleotide sequence according to a),
d) a nucleic acid molecule which, as a consequence of the genetic code, is degenerated into a nucleotide sequence according to a), and
e) a nucleic acid molecule in accordance with a nucleotide sequence according to a), which is modified and functionally analogous to a nucleotide sequence according to a) as a result of deletions, additions, substitutions, translocations, inversions and/or insertions.

The nucleic acid molecule according to SEQ ID NO 1 is preferably characterized in that the nucleotide sequence specified under c) has at least 40% homology to a nucleotide sequence specified under a).

The nucleic acid molecule according to SEQ ID NO 1 is preferably characterized in that the nucleotide sequence specified under c) has at least 60%, preferably 70%, more preferably 80%, especially preferably 90% homology to a nucleotide sequence specified under a).

The nucleic acid sequence or parts thereof can also be introduced into a different vector, which is preferably equivalent to pDBR, wherein the equivalent vector fulfils the same functions in the same way as the vector pDBR and also results in the same effect or wherein a person skilled in the arts would choose the equivalent vector in order to achieve the same effect as with the vector pDBR. One skilled in the art could produce the modified B cells using alternative vectors to those lentiviral vectors described in the present application.

The nucleic acid molecule described herein has at least 60%, preferably 70%, more preferably 80%, and most preferably 90% homology to a complementary nucleic acid molecule to a nucleic acids sequence according to SEQ ID NO 1 encoding an amino acid sequence, said nucleic acid molecule having a biological activity just like the nucleic acid molecule encoding a sequence in accordance with SEQ ID NO 1 or a complementary sequence thereof. In another preferred embodiment of the invention the nucleic acid molecule is genomic DNA, cDNA and/or RNA. Disclosed herein are also vectors and host cells and/or vaccines comprising the nucleic acid molecule.

Disclosed herein is an amino acid sequence and corresponding polypeptide encoded by the nucleic acid molecule and/or sequence according to SEQ ID NO 1, fragments or parts of SEQ ID NO 1 that preferably relate to open reading frames that code for functional proteins, especially proteins involved in function of the vector as described herein, and/or homologues thereof.

Also disclosed herein are amino acid sequences and corresponding polypeptide molecules that comprise an additional analogue amino acid or another structure (lipid and/or sugar residues).

According to the present disclosure, the amino acid sequence encoding a peptide is selected from the group comprising:
a) a peptide encoded by the nucleic acid sequence according to SEQ ID NO 1 or part thereof;
b) a peptide comprising an amino acid sequence having sufficient homology to be functionally analogous to an amino acid sequence in accordance with a);
c) a peptide according to an amino acid sequence a) or b) which is modified by deletions, additions, substitutions, translocations, inversions and/or insertions and functionally analogous to an amino acid sequence in accordance with a) or b).

The peptide which has sufficient homology to be functionally analogous to a peptide or amino acid sequence encoded by the nucleic acid sequence according to SEQ ID NO 1 or part thereof has at least 40% homology thereto.

The amino acid sequences have at least 60%, preferably 70%, more preferably 80%, especially preferably 90% homology to a peptide or amino acid sequence encoded by the nucleic acid sequence according to SEQ ID NO 1 or part thereof.

The invention further relates to the use of modified B cells according to the present invention for the induction of immune tolerance and/or the suppression and/or inhibition of immune reactions, preferably antigen-specific immune reactions directed against the antigen encoded by the exogenous nucleic acid and/or unwanted adaptive immune reactions mediated by CD4+ T cells, CD8+ T cells, B cells and/or antibodies. The modified B cells are engineered to express a selected antigen in such a way that at least one, preferably two, more preferably all three arms of the adaptive immune response, which react to any given specific antigen (CD4 T cells, CD8 T cells, or antibodies and B cells), are suppressed in a specific manner.

In a further embodiment the invention relates to the use of modified B cells according to the present invention for the suppression and/or inhibition of immune reactions, characterised in that the suppression of an immune reaction is associated with the inhibition and/or prevention of an inflammatory T cell response, preferably by suppression of IFN-γ-, IL-17- and/or TNF-α-producing CD4+ or CD8+ T cells, especially of TH1 and/or TH17 cell types.

The modified B cells of the present invention can be applied in the treatment of any unwanted immune reaction, especially:
- autoimmune disorders (in which it is known that certain antigen(s) have a key role as driver(s) of the disease, e.g. mysthenia gravis, a certain MS situation with a certain MHC allele),
- allergies,
- immune responses directed to proteins expressed in the course of gene therapy, and/or
- therapeutic proteins, such as hemophilia (factor VIII), enzyme replacement therapies, antibodies or cytokines.

Therefore the invention relates to the suppression of self-reactive humoral immune responses, such as autoimmune reactions, preferably by reducing the levels of antibodies directed against the antigen encoded by the exogenous nucleic acid.

The invention further relates to the use of modified B cells according to the present invention for the suppression and/or inhibition of immune reactions, characterised in that antigen-specific immune reactions, which are directed against an antigen expressed in the course of gene therapy, are suppressed.

This approach can be applied to suppress an immune response, especially to prevent immune reactions to specific proteins when their expression is restored by gene therapy in individuals with corresponding genetic deficiencies. For example the B cells of the present invention can be used to prevent immune reactivity towards protein normally absent in the patient due to stop mutations, while their reconstitution is achieved by gene therapy. For example, a gene therapy patient can be additionally treated with modified naive B cells that express a protein antigen, which is also the therapeutic gene product to be expressed via gene therapy. Through this approach the gene therapy product is less likely to be degraded by specific immune reactions upon expression in vivo.

Protein therapy is an area of medical innovation that is becoming more widespread, and involves the application of proteins, such as enzymes, antibodies or cytokines, directly to patients as therapeutic products. One of the major hurdles in delivery of such medicaments involves the immune responses directed against the therapeutic protein themselves. Administration of protein-based therapeutics is often accompanied by administration of immune suppressants, which are used in order to facilitate a longer lifetime of the protein and therefore increased uptake of the protein into the cells and tissues of the organism. General immune suppressants can however be disadvantageous due to the unspecific nature of the immune suppression that is carried out, resulting in unwanted side effects in the patient.

The present invention offers a method for suppression of immune responses against specific antigens. Therefore, a further aspect of the invention relates to the use of modified B cells according to the present invention for the suppression and/or inhibition of immune reactions targeted against therapeutic proteins and peptides, such as therapeutic antibodies, cytokines, enzymes or any other protein administered to a patient.

In a preferred embodiment the modified B cells according to the present invention are characterised in that an antigen-presenting molecule or co-stimulatory molecule involved in presentation of antigen, preferably MHC-II or its human counterpart HLA, is additionally modulated in the modified B cells. In a further embodiment, the antigen-presenting molecule or co-stimulatory molecule involved in presentation of antigen is inhibited, blocked, suppressed, activated or over-expressed in the B cells. For example, the administration of modified B cells in which MHC-II is inhibited or suppressed leads to significant autoimmune disease protection in mouse models. Therefore, the presentation of autoimmune disease causing epitopes via an alternative antigen presentation pathway (alternative to MHC-II) leads to the surprising suppression of an immune response against the epitope being presented. In light of this, the present invention relates additionally to the modulation, for example by suppression or enhancement, of antigen presentation molecules in combination with B cell application in order to control, enhance or regulate the immune suppression caused by the B cells of the present invention. The combinatorial regulation of multiple antigen presentation mechanisms provides a method for regulating the strength of the immune suppression initiated by the modified B cells.

Methods for the inhibition, blocking, suppression, activation or over-expression of an antigen-presenting molecule or co-stimulatory molecule involved in presentation of antigen are known to one skilled in the art. For example blocking or suppression of an antigen-presenting molecule or co-stimulatory molecule involved in presentation of antigen could be carried out via an antibody targeting the protein, genetic modification of the cell, such as deletions or other targeted mutations, or the use of anti-sense RNA approaches, such as siRNA, in order to silence the protein before translation of its mRNA. The activation or over-expression could be achieved via administration of activating antibodies, the administration of active protein to the cell or by over expression of the particular protein in an expression vector, or by transformation of any other exogenous nucleic acid coding for the protein to be increased.

A further aspect of the invention relates to a pharmaceutical composition comprising modified B cells of the present invention and at least one pharmaceutically acceptable carrier.

A further aspect of the invention relates to the modified B cells of the present invention for use as a medicament in the treatment of unwanted immune reactions, such as those involved in autoimmune disorders, immune reactions to therapeutic proteins, and/or allergies.

One aspect of the invention relates to the use of modified B cells according to the present invention in the manufacture of a pharmaceutical composition for use in the treatment of autoimmune disorders or allergies.

A further aspect of the invention relates to modified B cells, characterised in that the B cells are obtainable by isolation of non-activated B cells, preferably by isolation of CD19+ cells, followed by transformation of the isolated B cells with a lentiviral vector, which encodes the antigen to be presented.

A further aspect of the invention relates to a method for the suppression and/or inhibition of immune reactions, preferably antigen-specific immune reactions directed against the antigen encoded by the exogenous nucleic acid and/or unwanted adaptive immune reactions mediated by CD4+ T cells, CD8+ T cells, B cells and/or antibodies, characterised in that a therapeutically effective amount of modified B cells according to the present invention are administered to a mammal, preferably animal or human, patient.

The molecule names listed in the description (such as interleukins, cytokines, cluster of differentiation proteins, antigen presenting molecules, amongst others) refer to the molecule names in the mouse. Considering the present invention can be applied to human B cells and their use in humans, the human counterparts of such molecules apply, for example HLA which corresponds to MHC-II in mouse, and are thus included within the scope of the invention.

### DETAILED DESCRIPTION OF THE INVENTION

The term "nucleic acids" refer a nucleic acid DNA or RNA molecule, either single or double-stranded, as is commonly understood by one skilled in the art. Preferred nucleic acids of the present invention refer to DNA expression vectors, plasmids, viral vectors, lentiviral vectors, HIV-based lentiviral vectors, RNA molecules or more specifically mRNA molecules.

The term "exogenous nucleic acids" refers to any nucleic acid that does not originate from the cell into which it has been transformed. The nucleic acid sequence may be contained within the genome of the organism into which the nucleic acid molecule is transformed. However, the nucleic acid molecule itself must be transformed into the target cells, thereby originating from an external source. Such exogenous nucleic acids may be synthetic in nature, be produced recombinantly or be purified or extracted from other organisms, or stem from any other source external to the cell to be transformed. In the present invention the exogenous nature does not necessarily refer to the sequence of the nucleic molecule but to its origin. The molecule which is then encoded by the nucleic acid can therefore be a protein or peptide also encoded for by the genome of the recipient cell.

The introduction of exogenous nucleic acids can be carried out via transfection, transduction, transformation or any other process of genetic modification or transformation. This can take place naturally, as occurs when a virus infects cells, or artificially. Methods of artificial transfection include but are not limited to chemical methods, including calcium phosphate precipitation, DEAE-dextran complexation and lipid-mediated DNA transfer; physical methods, including electroporation, microinjection, and biolistic particle delivery (gene gun); and using recombinant, lab manipulated viruses as vectors.

The term "transformation" relates to the bringing of exogenous nucleic acids into the cell via either natural or chemical methods.

The terms "inactivated B cells", non-activated B cells, "resting B cells", "unstimulated B cells", "quiescent B cells", or "naive B cells" refer to any B cell not activated especially not deliberately activated by an antigen or via B-cell receptors, toll-like receptors and/or CD40 receptors. The invention preferably relates to B cells that were purified and modified directly without any prior exposure to any stimulus of B cell activation.

The term "reprogrammed B cells" refers to B cells that have been transformed or modified with a nucleic acid that encodes an antigen to be presented.

The term "resting regulatory B cells" refers to quiescent B cells that possess one, two or more of the suppressive properties of activated B cells, such as IL-10 secretion.

An "autoimmune disorder" herein is a disease or disorder arising from and directed against an individual's own tissues or a co-segregate or manifestation thereof or resulting condition therefrom. Examples of autoimmune diseases or disorders include, but are not limited to arthritis (rheumatoid arthritis, juvenile-onset rheumatoid arthritis, osteoarthritis, psoriatic arthritis, and ankylosing spondylitis), psoriasis, dermatitis including atopic dermatitis, chronic idiopathic urticaria, including chronic autoimmune urticaria, polymyositis/ dermatomyositis, toxic epidermal necrolysis, scleroderma (including systemic scleroderma), sclerosis such as progressive systemic sclerosis, inflammatory bowel disease (IBD) (for example, Crohn's disease, ulcerative colitis, autoimmune inflammatory bowel disease), pyoderma gangrenosum, erythema nodosum, primary sclerosing cholangitis, episcleritis), respiratory distress syndrome, including adult respiratory distress syndrome (ARDS), meningitis, IgE-mediated diseases such as anaphylaxis and allergic and atopic rhinitis, encephalitis such as Rasmussen's encephalitis, uveitis or autoimmune uveitis, colitis such as microscopic colitis and collagenous colitis, glomerulonephritis (GN) such as membranous GN (membranous nephropathy), idiopathic membranous GN, membranous proliferative GN (MPGN), including Type I and Type II, and rapidly progressive GN, allergic conditions, allergic reaction, eczema, asthma, conditions involving infiltration of T cells and chronic inflammatory responses, atherosclerosis, autoimmune myocarditis, leukocyte adhesion deficiency, systemic lupus erythematosus (SLE) such as cutaneous SLE, subacute cutaneous lupus erythematosus, lupus (including nephritis, cerebritis, pediatric, non-renal, discoid, alopecia), juvenile onset (Type I) diabetes mellitus, including pediatric insulin-dependent diabetes mellitus (IDDM), adult onset diabetes mellitus (Type II diabetes), multiple sclerosis (MS) such as spino-optical MS, immune responses associated with acute and delayed hypersensitivity mediated by cytokines and T-lymphocytes, tuberculosis, sarcoidosis, granulomatosis including lymphomatoid granulomatosis, Wegener's granulomatosis, agranulocytosis, vasculitis (including large vessel vasculitis (including polymyalgia rheumatica and giant cell (Takayasu's) arteritis), medium vessel vasculitis (including Kawasaki's disease and polyarteritis nodosa), CNS vasculitis, systemic necrotizing vasculitis, and ANCA-associated vasculitis, such as Churg-Strauss vasculitis or syndrome (CSS)), temporal arteritis, aplastic anemia, Coombs positive anemia, Diamond Blackfan anemia, hemolytic anemia or immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, CNS inflammatory disorders, multiple organ injury syndrome, antigen-antibody complex mediated diseases, anti-glomerular basement membrane disease, antiphospholipid antibody syndrome, allergic neuritis, Bechet's or Behcet's disease, Castleman's syndrome, Goodpasture's syndrome, Reynaud's syndrome, Sjogren's syndrome, Stevens-Johnson syndrome, pemphigoid such as pemphigoid bullous, pemphigus (including vulgaris, foliaceus, and pemphigus mucus-membrane pemphigoid), autoimmune polyendocrinopathies, Reiter's disease, immune complex nephritis, chronic neuropathy such as IgM polyneuropathies or IgMmediated neuropathy, thrombocytopenia (as developed by myocardial infarction patients, for example), including thrombotic thrombocytopenic purpura (TTP) and autoimmune or immune-mediated thrombocytopenia such as idiopathic thrombocytopenic purpura (ITP) including chronic or acute ITP, autoimmune disease of the testis and ovary including autoimune orchitis and oophoritis, primary hypothyroidism, hypoparathyroidism, autoimmune endocrine diseases including thyroiditis such as autoimmune thyroiditis, chronic thyroiditis (Hashimoto's thyroiditis), or subacute thyroiditis, autoimmune thyroid disease, idiopathic hypothyroidism, Addison's disease, Grave's disease, polyglandular syndromes such as autoimmune polyglandular syndromes (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis such as allergic encephalomyelitis, myasthenia gravis, cerebellar degeneration, limbic and/or brainstem encephalitis, neuromyotonia, opsoclonus or opsoclonus myoclonus syndrome (OMS), and sensory neuropathy, Sheehan's syndrome, autoimmune hepatitis, chronic hepatitis, lupoid hepatitis, chronic active hepatitis or autoimmune chronic active hepatitis, lymphoid interstitial pneumonitis, bronchiolitis obliterans (non-transplant) vs NSIP, GuillainBarre syndrome, Berger's disease (IgA nephropathy), primary biliary cirrhosis, celiac sprue (gluten enteropathy), refractory sprue, dermatitis herpetiformis, cryoglobulinemia, amyotrophic lateral sclerosis (ALS; Lou Gehrig's disease), coronary artery disease, autoimmune inner ear disease (AIED) or autoimmune hearing loss, opsoclonus myoclonus syndrome (OMS), polychondritis such as refractory polychondritis, pulmonary alveolar proteinosis, amyloidosis, giant cell hepatitis, scleritis, a non-cancerous lymphocytosis, a primary lymphocytosis, which includes monoclonal B cell lymphocytosis (e.g., benign monoclonal gammopathy and monoclonal gammopathy of undetermined significance, MGUS), peripheral neuropathy, paraneoplastic syndrome, channelopathies such as epilepsy, migraine, arrhythmia, muscular disorders, deafness, blindness, periodic paralysis, and channelopathies of the CNS, autism, inflammatory myopathy, focal segmental glomerulosclerosis (FSGS), endocrine ophthalmopathy, uveoretinitis, autoimmune hepatological disorder, fibromyalgia, multiple endocrine failure, Schmidt's syndrome, adrenalitis, gastric atrophy, presenile dementia, demyelinating diseases, Dressler's syndrome, alopecia areata, CREST syndrome (calcinosis, Raynaud's phenomenon, esophageal dysmotility, sclerodactyly, and telangiectasia), male and female autoimmune infertility, ankylosing spondolytis, mixed connective tissue disease, Chagas' disease, rheumatic fever, recurrent abortion, farmer's lung, erythema multiforme, post-cardiotomy syndrome, Cushing's syndrome, bird-fancier's lung, Alport's syndrome, alveolitis such as allergic alveolitis and fibrosing alveolitis, interstitial lung disease, transfusion reaction, leprosy, malaria, leishmaniasis, kypanosomiasis, schistosomiasis, ascariasis, aspergillosis, Sampter's syndrome, Caplan's syndrome, dengue, endocarditis, endomyocardial fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, or Fuch's cyclitis, Henoch-Schonlein purpura, human immunodeficiency virus (HIV) infection, echovirus infection, cardiomyopathy, Alzheimer's disease, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, and giant cell polymyalgia. The treatment of autoimmune disorders via immune suppression is therefore one embodiment of the present invention.

As used herein, the term "allergy" or "allergies" refers to a disorder (or improper reaction) of the immune system. Allergic reactions occur to normally harmless environmental substances known as allergens; these reactions are acquired, predictable, and rapid. Strictly, allergy is one of four forms of hypersensitivity and is called type I (or immediate) hypersensitivity. It is characterized by excessive activation of certain white blood cells called mast cells and basophils by a type of antibody known as IgE, resulting in an extreme inflammatory response.

Common allergic reactions include eczema, hives, hay fever, asthma, food allergies, and reactions to the venom of stinging insects such as wasps and bees. Mild allergies like hay fever are highly prevalent in the human population and cause symptoms such as allergic conjunctivitis, itchiness, and runny nose. Allergies can play a major role in conditions such as asthma. In some people, severe allergies to environmental or dietary allergens or to medication may result in life-threatening anaphylactic reactions and potentially death. The treatment of allergies via immune suppression is therefore one embodiment of the present invention.

As used herein, the term "gene therapy" relates to the introduction and expression in an animal (preferably a human) of an exogenous sequence (e.g.; a therapeutic gene or cDNA sequence coding for a therapeutic protein, for example a full and functional sequence for a gene, for which the endogenous gene of the animal or human contains a mutation) to supplement, replace or inhibit a target gene, or to enable target cells to produce a protein that has a prophylactic or therapeutic effect toward any given condition or illness.

The term "protein therapy", "protein therapeutics" or "therapeutical proteins" refer to proteins and/or peptides and their administration in the therapy of any given condition or illness. Therapeutical proteins relate to any proteins or peptides, such as therapeutic antibodies, cytokines, enzymes or any other protein, that is administered to a patient. Examples of protein therapy relate to treatment of hemophilia via administration of plasma-derived or recombinant clotting factor concentrates, the treatment of cancer or cardiovascular disease using monoclonal antibodies or the treatment of metabolic or lysosomal disease by enzyme replacement therapy.

As used herein "pharmaceutical composition" means a composition comprising a B cell preparation and at least one ingredient that is not an active ingredient whereby the composition can be safely and effectively used as a product to obtain or achieve a desired outcome. The term "pharmaceutical composition" as used herein means compositions which result from the combination of individual components which are themselves pharmaceutically acceptable. For example, where intravenous administration is foreseen, the components are suitable or acceptable (in both quality and quantity) for intravenous administration. The B cells of the present invention can be administered to mammals, namely humans and livestock, by numerous routes, such as intravenously, subcutaneously or intramuscularly. The dose administered may be between about 1,000 and about 1000,000,000 cells per dose, or other amounts understood by a person of ordinary skill in the art to be therapeutically effective as a therapy to treat or prevent symptoms of unwanted immune reactions.

The disclosure presented herein is directed towards a pharmaceutical composition which can be administered through a variety of routes including intravenously, subcutaneously, intramuscularly or directly into or onto the affected individual. When the pharmaceutical composition is delivered via an injection, the injection of the B cell composition can occur as a single injection or multiple injections at any location inside or outside the body and the injection(s) can occur in a single day or over multiple days. The daily dose is administered to a subject wherein the daily amount of the B cell preparation delivered to the subject from the pharmaceutical composition is about that which is therapeutically effective for treating symptoms associated with unwanted immune reactions. Additionally, the pharmaceutical composition may optionally include additional components such as salts, stabilizers and antimicrobials without departing from the spirit and scope of the claimed invention. The pharmaceutical composition of the present invention contains a B cell preparation and a pharmaceutically acceptable carrier.

The quantity and nature of the B cells to be incorporated in the composition will vary depending on desired therapeutic effect and the time span for which the composition is to provide a therapeutic effect. The quantity of B cells in the pharmaceutical composition is that which will deliver a therapeutically effective amount for treating symptoms associated with unwanted immune reactions. Of course, the concentration and character of the B cells to be included in the pharmaceutical composition will vary depending upon the components used in the composition, the route by which it is administered, the symptoms and details of the immune reaction which requires treatment as well as other factors known to those of skill in the art.

Although the invention has been described with respect to specific embodiments and examples, it should be appreciated that other embodiments utilizing the concept of the present invention are possible without departing from the scope of the claims.

### EXAMPLES

### Example 1. Effective engineering of unstimulated quiescent B cells by lentiviral transduction

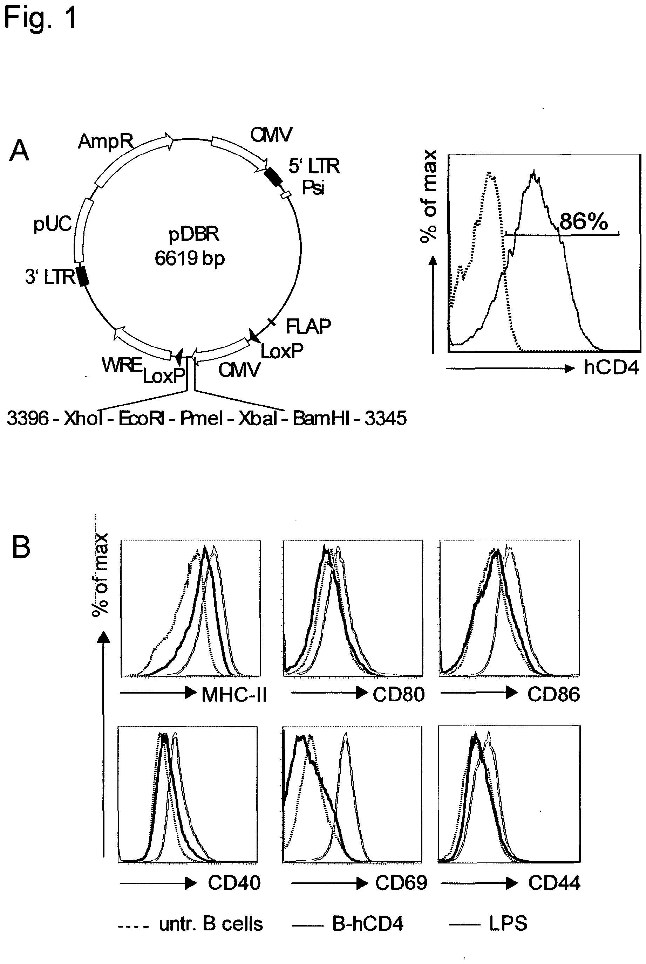

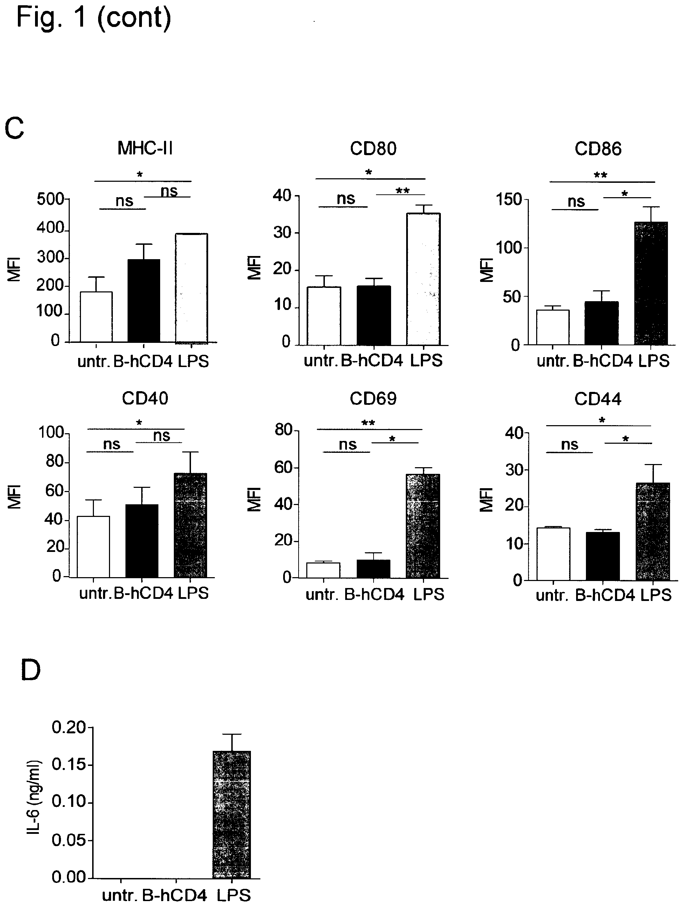

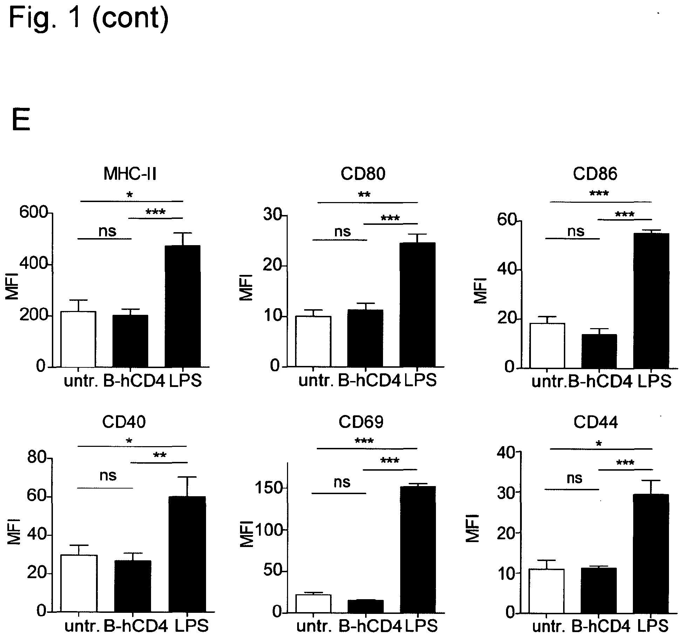

A protocol for genetic engineering of resting mouse B cells was developed using a lentiviral vector coding for human CD4 (hCD4) (pDBR) (Fig. 1). Remarkably, it was sufficient to centrifuge purified B cells in presence of lentiviral particles for 75 minutes to obtain very high transduction efficiency. After centrifugation, B cells were extensively washed and incubated for 18h in regular medium to allow for expression of the reporter gene. Representation of the level of hCD4 measured at this time point by flow cytometry revealed a bell-shaped histogram suggesting that all the B cells were transduced and expressed hCD4 (Fig. 1a). Thus, resting B cells can be transduced very efficiently using HIV-based lentiviral vectors, in absence of any activating agent or cytokine. However, it is also possible to use equivalent vectors achieving similar or identical results.

B cells are equipped with various pathogen recognition receptors, including TLR that can sense viral nucleic acids (17507480, 15778362, 19587008, 9237759). The triggering of such receptors usually leads to robust cellular activation, up-regulation of co-stimulatory molecules such as CD80 and CD86, and it can overcome the tolerogenic properties of resting B cells (2700931, 1730913). It was therefore assessed how lentiviral transduction affected the expression of co-stimulatory molecules by B cells. At 18h after transduction, B cells transduced with pDBR (B-hCD4 cells) displayed similar levels of MHC-II, CD40, CD44, CD69, CD80, and CD86 as naive B cells, and they did not produce any IL-6, an essential cytokine for the activation of immunity (Fig. 1b,c). In contrast, B cells cultured in presence of the TLR-4 agonist lipopolysaccharides displayed an activated phenotype, and produced IL-6 (Fig. 1b,c). Thus, resting B cells retain a quiescent phenotype during their genetic re-programming. This example demonstrates the design of "resting regulatory B cells" combining the weak immunogenicity of resting B cells with the effective suppressive functions of activated B cells. The modified B cells preserve especially a resting phenotype following administration into patients. It has never been shown before, that B cell modified with lentiviral vectors maintain a resting or naive phenotype following administration in recipient organisms.

### Example 2. Engineering of antigen-presenting and IL-10-secreting quiescent B cells through lentiviral transduction

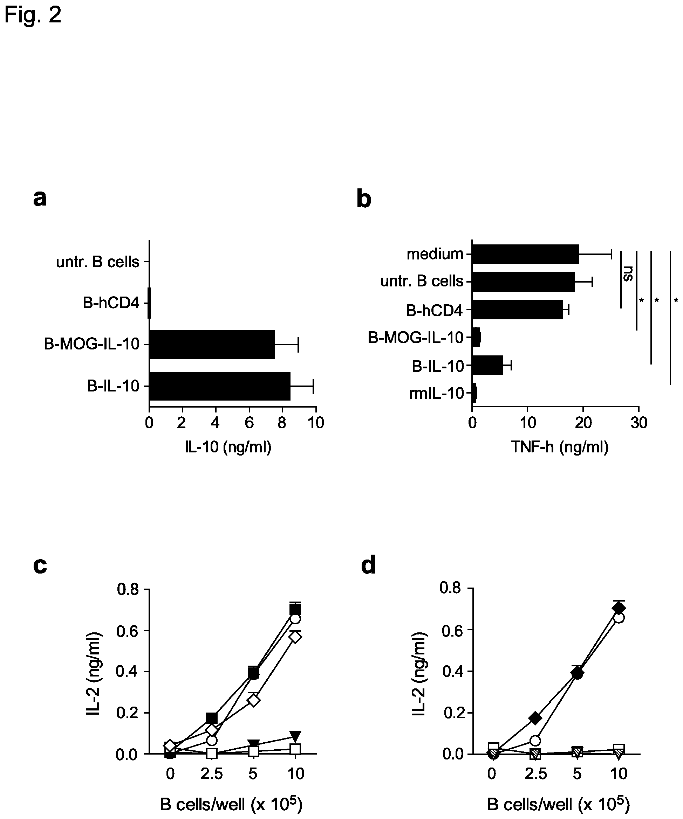

B cells were transduced as described above with a lentiviral vector containing the sequence coding for mouse IL-10 (pDBR-IL-10). Some B cells were additionally transduced with a lentiviral vector carrying the sequence coding for full-length mouse myelin oligodendrocyte glycoprotein (MOG) (pDBR-MOG). Culture supernatants from B cells transduced with pDBR-IL-10 (B-IL-10 cells), or both pDBR-MOG and pDBR-IL-10 (B-MOG-IL-10 cells) contained around 8 ng/ml IL-10, while this cytokine was undetectable in supernatants from B cells transduced with pDBR-hCD4 (B-hCD4 cells) (Fig. 2a). To evaluate the activity of this B cell-derived IL-10, the supernatants produced by the various transduced B cells were tested for their capacity to inhibit TNF-α production by CpG-activated bone marrow-derived macrophages. Supernatants produced by untransduced B cells or by B-hCD4 cells, which did not contain any IL-10, did not have any effect on this response (Fig. 2b). In contrast, supernatants from B-IL-10 or B-MOG-IL-10 cells, which both contained IL-10, markedly inhibited TNF-α production by CpG-activated macrophages, similarly to recombinant IL-10 (Fig. 2b). Thus, quiescent B cells can be programmed to produce functionally active immunosuppressive cytokines by lentiviral transduction.

It was then demonstrated that this approach allowed designing of resting B cells capable of presenting antigen to specific CD4 T cells. For this, B cells were transduced with pDBR-MOG (B-MOG cells). After centrifugation and washing, B-MOG cells were immediately cultivated with the MOG-reactive T cell hybridoma P32.2, which secretes IL-2 upon stimulation with MOG-MHC-II complexes (16506290). Supernatants from co-cultures comprising P32.2 and either B-MOG or B-MOG-IL-10 cells contained IL-2, suggesting that these B cells presented MOG-derived epitopes via MHC-II (Fig. 2c). Indeed, the IL-2 response was abolished in co-cultures supplemented with a blocking anti-MHC-II antibody (Fig. 2d). Thus, quiescent B cells can be engineered to present antigen to MHC-II-restricted CD4 T cells. Examination of co-stimulatory receptors expression by B-MOG cells, B-IL-10 cells, and B-MOG-IL-10 cells confirmed that they expressed a resting phenotype similarly to B-hCD4 cells. This example demonstrates that quiescent B cells can be reprogrammed to acquire molecular features involved in the suppressive properties of activated B cells while maintaining a resting phenotype.

### Example 3. Reprogrammed quiescent B cells provide full protection from chronic EAE

The suppressive function of modified quiescent B cells were evaluated in EAE, a T cell-mediated demyelinating and paralyzing autoimmune disease that serves as a model for multiple sclerosis. EAE can be induced in mice by immunization with MOG emulsified in adjuvant, which leads to the development of inflammatory lesions in brain and spinal cord, and subsequent paralysis.

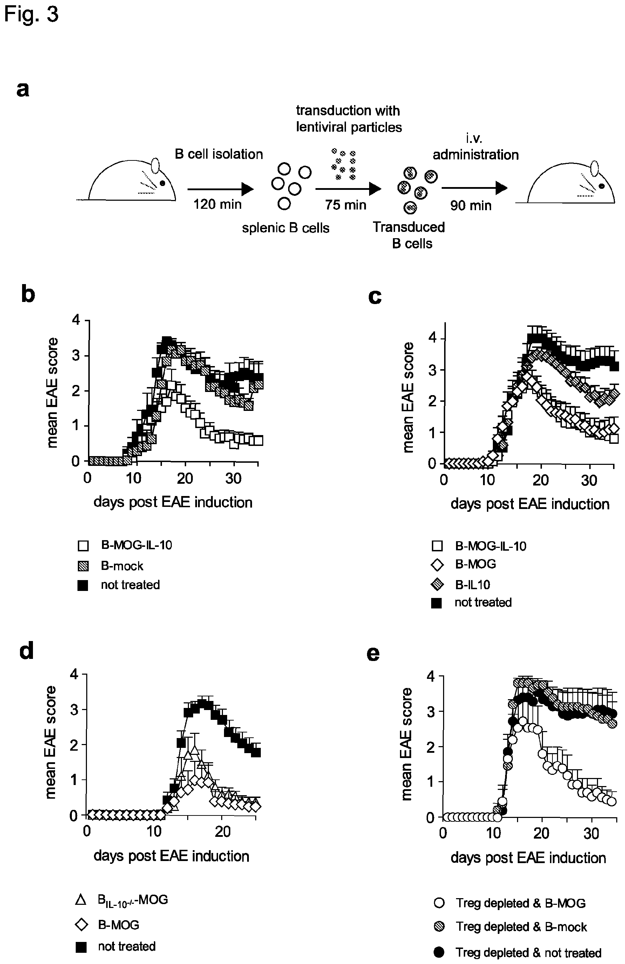

It was asked whether B-MOG-IL-10 cells, engineered to present MOG and secrete IL-10, could suppress EAE when administered in two doses on days -6 and -2 before immunization (Fig. 3a). Indeed, this treatment resulted in reduced disease severity and improved recovery of recipient mice, compared to mice treated with control B cells or untreated mice (Fig. 3b). To identify the respective contributions of IL-10 and MOG to this protective effect, mice were vaccinated with B cells transduced with pDBR-IL-10 alone (B-IL-10 cells), or pDBR-MOG alone (B-MOG cells). B-MOG cells protected recipient mice as effectively as B-MOG-IL-10 cells (Fig. 3c). In contrast, B-IL-10 cells had no therapeutic effect (Fig. 3c). To address further the role of IL-10, the protective effects of B-MOG cells prepared by transduction with pDBR-MOG of either wild-type or IL-10-deficient B cells (Fig. 3d) were compared. The two types of B cells led to comparable amelioration of disease. It was concluded from these experiments that resting B cells engineered to express MOG can provide protection from EAE, with no beneficial effect added by IL-10 expression. Therefore all following experiments were performed with B-MOG cells.

In all experiments B-MOG cells induced a more effective remission from paralysis. Therefore it was examined how this therapeutic approach performed in an experimental condition normally leading to chronic disease. Natural regulatory CD4⁺CD25⁺Foxp3⁺ T cells (Treg) are critical for the recovery from EAE, and they are impaired in patients with MS (16116190, 20159585). As expected, administration of an anti-CD25 antibody to mice prior to immunization, which is known to impair Treg numbers and functions, resulted in a severe chronic form of EAE (Fig. 3e). Remarkably, adoptive therapy with B-MOG cells protected recipient mice from this non-remitting form of EAE, allowing a full recovery from the disease by day 35 post-immunization (Fig. 3e).

This example demonstrates that reprogrammed resting B cells can suppress EAE, and remarkably, provide full protection from the chronic disease that would otherwise develop when Treg cells are defective. Considering EAE is an animal model for multiple sclerosis (MS), the present invention relates additionally to application in humans and other mammals.

### Example 4. Reprogrammed quiescent B cells suppress T cell- and B cell-mediated immunity

In order to identify how B-MOG cells facilitate remission from EAE, autoreactive T and B cell responses, which drive this disease, were monitored at different time points after immunization in mice treated or not with such B cells (3155574, 10556797).

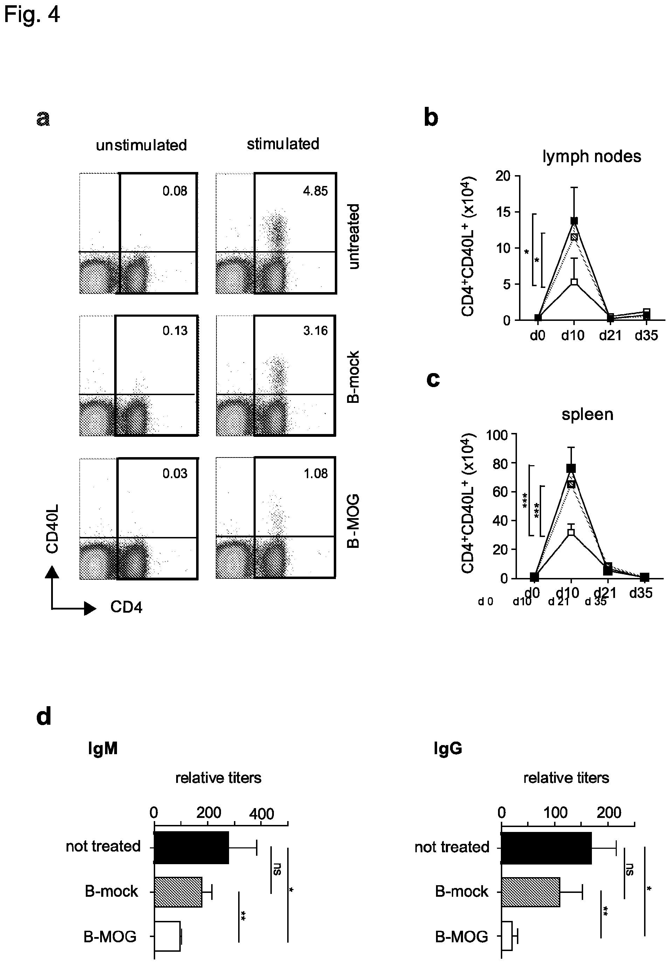

Autoreactive T helper cells are identifiable in a specific manner according to up-regulation of CD154 (CD40L) upon antigenic re-stimulation (Fig. 4a) (16186818). Following immunization, MOG-reactive CD4 T helper cells transiently accumulated in draining lymph nodes (dLN) and spleen, reaching maximum numbers at day 10 (Figs. 4b,c). Treatment with B-MOG cells markedly reduced (by approximately 50%) this response, while B-mock cells (B cells transduced with an "empty" pDBR vector) had little effect. An important function of CD4 T helper cells is the provision to B cells of signals required for humoral immunity. Mice treated with B-MOG cells displayed decreased levels of MOG-reactive IgM and IgG autoantibodies in serum at day 21 post-immunization, compared to untreated mice or mice treated with B-mock cells (Fig. 4d). There the present invention offers a method to suppress the production of specific antibodies in treated individuals or mammals.

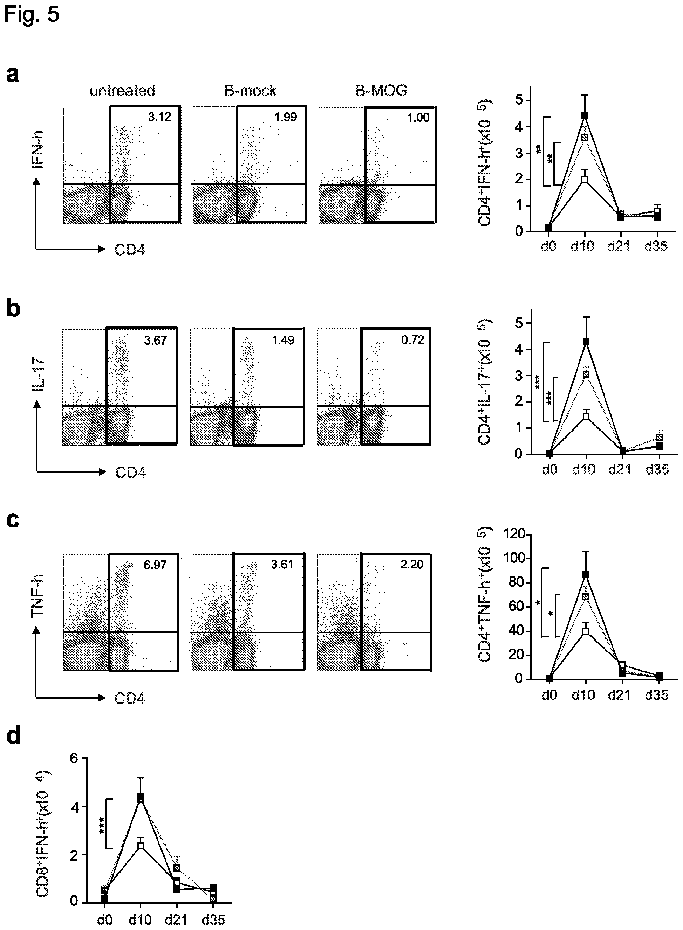

The encephalitogenic function of autoreactive T cells is tightly associated to their secretion of inflammatory cytokines (7691946, 16200068). The cytokine-producing CD4 and CD8 T cells were enumerated by flow cytometry after re-stimulation of spleen cells with MOG(35-55) for 6h ex *vivo* (Figs 5a-c). CD4 T cells expressing IFN-γ, IL-17, and TNF-α□ transiently accumulated in spleen after immunization, reaching a peak at day 10 (Figs 5a-c). B-MOG cells markedly suppressed this response. They similarly inhibited IFN-γ production by CD8 T cells (Fig. 5d). This demonstrates that re-reprogrammed quiescent B cells can suppress adaptive immune responses mediated by CD4 T cells, CD8 T cells, antibodies and B cells upon adoptive transfer.

### Example 5. Reprogrammed quiescent B cells inhibit the accumulation of inflammatory cells in CNS

The development of EAE is associated to the infiltration of lymphocytes and mononuclear phagocytes in the CNS, where they cause inflammatory lesions. Therefore it was examined whether B-MOG cells inhibited EAE by modulating the accumulation of leukocytes in CNS.

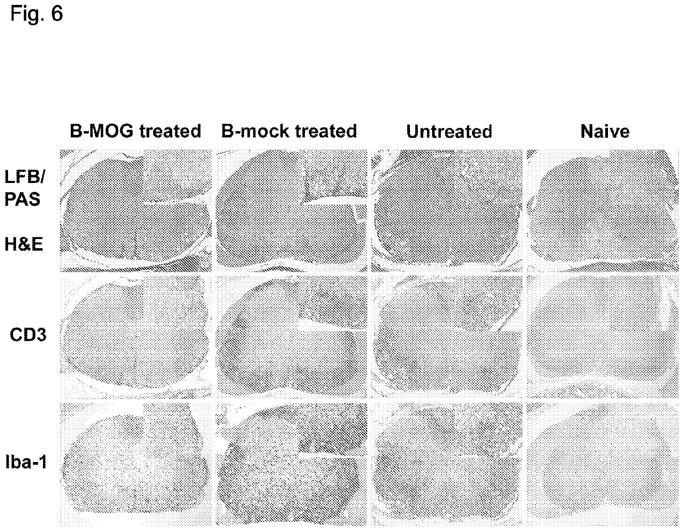

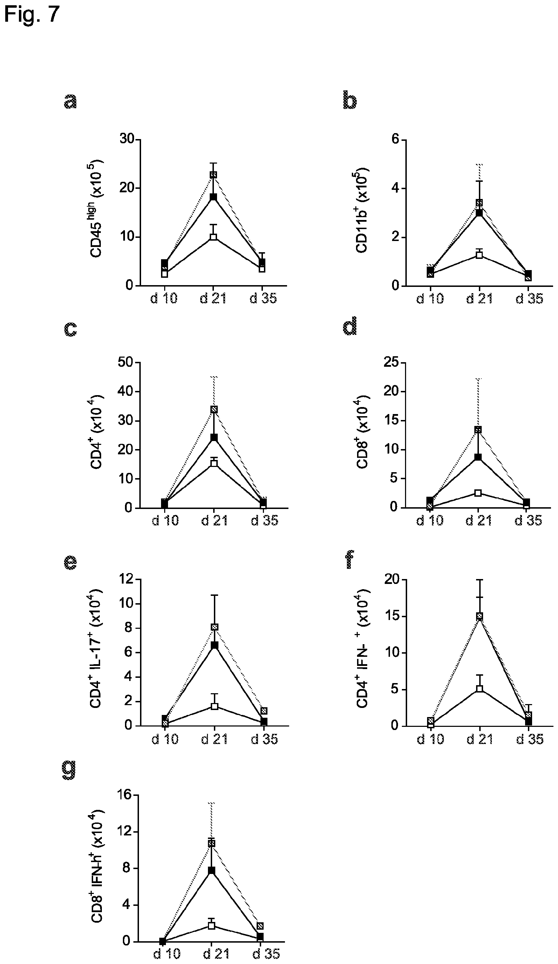

Histological analysis of spinal cord sections from treated and control mice revealed that recipients of B-MOG cells had lower numbers of infiltrating hematopoietic cells, including T cells and macrophages, at day 21 post EAE induction (Fig. 6). To quantify this effect, the CNS infiltrate of the various types of mice were analyzed by flow cytometry at disease onset (day 10), peak of disease (day 21), and after recovery from EAE (day 35). The numbers of CD45^{high} hematopoietic cells per brain reached maximum values at day 21 after EAE induction, which corresponded to the period of maximal disease severity for untreated mice. At this time, mice treated with B-MOG cells had about two times less hematopoietic CD45^{high} cells in brain than control mice (Fig. 7a). This was associated with reduced numbers of CD11b⁺ myeloid cells, as well as CD4⁺and CD8⁺T cells (Figs. 7b-d). To characterize the functional properties of the infiltrating T cells in mice treated with B-MOG cells and in control mice, isolated cells were re-stimulated with anti-CD3 and anti-CD28 for 6h, and stained for intra-cellular IFN-γ and IL-17. Mice treated with B-MOG cells had approximately 3 fold less inflammatory CD4 T cells of T_{H}1 and T_{H}17 types in brain than control mice (Figs. 7e,f). Consistent with this, they also had around 3 fold less IFN-γ-producing CD8 T cells (Fig. 7g). This example demonstrates that B-MOG cells prevent the accumulation of pathogenic cells in the target tissue. The B cell treatment also prevents tissue degradation by the immune attack, as shown by preservation of myelin integrity in luxol fast blue (LFB) staining (Figure 6).

### Example 6. Reprogrammed quiescent B cells do not influence Treg activation

B cells can regulate immunity by increasing Treg function. Although B-MOG cells restored full remission from EAE in mice lacking functional Treg cells (Fig. 3e), which highlights Treg-independent mechanisms of suppression, it was further examined whether B-MOG cells promoted Treg function in wild-type mice. Such an effect was tested by monitoring Treg numbers and proliferation in mice treated with B-MOG cells and in control mice at different time points after EAE induction.

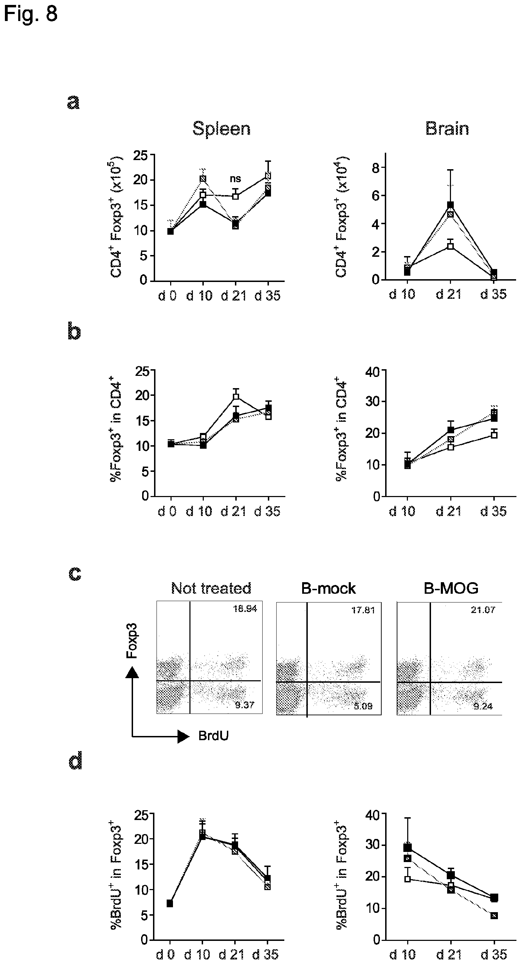

B-MOG cells did not significantly influence the numbers of Tregs in spleen (Fig. 8a). B-MOG cells also did not increase the number of Treg cells in brain (Fig. 8a). In fact, mice treated with B-MOG cells had fewer Tregs in brain at day 21, probably reflecting their milder local inflammation (Fig. 8a). It is generally assumed that the suppressive activity of Tregs is related to their frequency among CD4 T cells (16785514). The frequencies of Treg among CD4 T cells were similar in spleen and brain in the three groups of mice at different time points during EAE (Fig. 8b). It was further asked whether B-MOG cells affected the proliferation of Treg by performing *in vivo* bromodeoxyuridin incorporation analyzes (Fig. 8c). B-MOG cells had no effect on the percentage of proliferating Tregs, which increased rapidly and reached maximum values at day 10 after immunization in spleen and brain (Fig. 8d). This demonstrates that B-MOG cells do not influence Tregs activation during EAE, as assessed by cell numbers and proliferation. Taken together with the observation that B-MOG cells restored complete remission from disease in mice lacking functional Tregs, these findings demonstrated that B-MOG cells regulate immunity independently of Tregs.

### Example 7. Treatment with pDBR-MOG-tranduced MHC-II-deficient B cells

Antigen presentation can occur through various pathways. Different antigen-presentation pathways may lead to different immune modulation effects. Protection from EAE in mice was therefore tested using MHC-II knock-out modified B cells (MHC-IIko).

C57BL/6 mice received between 8 and 10 x 10⁶ tranduced MHC-IIko-B cells in 200µl of PBS i.v. on days -6 and -2 before EAE induction. One group of mice received MHC-II knock-out (ko) B cells transduced with pDBR-MOG (MHC-IIko-B-MOG) and another group of mice received MHC-IIko B cells transduced with empty pDBR (MHC-IIko B-mock). A third group of mice did not receive B cells. On day 0, EAE was induced by immunization with 50µg MOG (35-55) in CFA. 240ng of Pertussis Toxin per mouse was injected on day 0, and day +2. Mice were monitored for signs of disease.These data show that MHC-II ko B cells protect mice from EAE more effectively than WT B cells. The protection remains dependent on expression of MOG. Taken together, it was demonstrated that MOG is presented via a non-MHC-II molecule and thereby induces strong suppression of the immune response.

### MATERIALS AND METHODS USED IN THE EXAMPLES

### Mice and immunization

C57BL/6 and IL-10^{-/-} mice were bred under specific pathogen free conditions at the Bundesinstitut für Risikobewertung (Berlin, Germany). EAE was induced by immunization with MOG(35-55) peptide (Charité-Berlin) as previously described (12244307). All mice were used at 6-10 weeks of age. Animals were treated according to the requirements of the German legislation.

### Lentiviral vector construction, production of viral supernatants and B cell transduction.

Mouse MOG, mouse IL-10, and human CD4 (hCD4) coding sequences were cloned and inserted downstream of the CMV promoter of the lentiviral expression plasmid pDBR. MOG (pDBR-MOG), IL-10 (pDBR-IL-10), hCD4 control (pDBR-hCD4) and pDBR-empty construct (pDBR-mock) particles were generated by transient transfection of 293 T cells by using the three plasmid system (Pax2 packaging plasmid and vesicular stomatitis virus protein G encoded in the YITP plasmid). Viral supernatants were harvested 48 hours after transfection and supplemented with Hepes buffer (Gibco-Invitrogen) at 10mM and with hexadimethrine bromide at 10µg/ml (Sigma-Aldrich) before use. For B cell transduction, purified B cells were centrifuged at 1800 rpm during 75 min at room temperature with 3 ml of viral particle-containing- supernatants. After this time, the cells were washed in complete RPMI medium and resuspended in complete RPMI medium for culture. Transduced B cells were resuspended in PBS for mouse i.v administration. Any antigen coding sequence can easily be cloned into pDBR. The modification of the vector with the antigen-encoding nucleic acid molecule can occur via methods known to one skilled in the art, such as recombinant DNA technology, involving for example standard cloning techniques such as restriction digestion and ligation or recombination-based approaches used to insert the antigen-coding DNA.

The vector pDBR according to SEQ ID NO 1 comprises the following sequence:

### B Cell purification and adoptive transfers

Splenic B cells were obtained using anti-CD43 microbeads (Miltenyi Biotec). Purity was above 97% according to CD19 expression. After transduction, 1x10⁷ B cells were administered intravenously to recipient C57BL/6 mice.

### Cytokine ELISA

Microtiter plates (high binding EIA/RIA plates, Costar) were pre-coated with an anti-IL-10 (SXC1), -IL-6 (20/F3), or-IL-2 (JES6-1A12) capture antibody. Culture supernatants were added for 1 hour at room temperature, followed by extensive washing. Detection antibodies were biotin-anti-IL-10 (JES5-2A5-7), -IL-6 (MP5-32C11) or -anti-IL-2 (JES6-5H4) were then incubated, and ELISA was developed as previously described (12244307).

### MOG35-55-specific antibody measurement in blood serum

Blood was obtained from the lateral vein tail on day 21 and serum was prepared. For determination of MOG(35-55)-specific Abs, 96-well microtiter plates (high binding EIA/RIA plates) were coated with MOG(35-55) (10µg/ml) at 4°C overnight. Plates were washed with PBS and blocked with PBS and 1 % BSA at 4°C. Plates were washed again, serial dilutions of sera were added and plates were incubated at room temperature for 2 h. Plates were washed 3 times with PBS and 0.1% Tween 20 and incubated with alkaline phosphatase- conjugated mAb (1µg/ml in PBS and 0.05% Tween 20) goat anti-mouse anti-lgM-AP (Southern Biotech) and goat anti-mouse IgG (Southern Biotech) were incubated for 1h at room temperature, washed 3 times with PBS and 0.1% Tween 20, and developed by the addition of 50 µl of p-nitrophenyl phosphate (PNPP; Sigma) substrate (1 mg/ml in diethanolamine buffer, pH 9.8).and absorption at 405 nm was determined.

### Analysis of transduced cells

Transduction efficiency was checked on 293 T cells and on B cells, 24 hours after transduction by flow cytometric analysis of hCD4 expressing cells. The cells were stained with cy54-anti-humanCD4 Ab (TT1) and bio-anti-CD19, plus PE-streptavidin. Efficiency of MOG transduction was checked by culturing transduced B cells with P32.2 MOG-specific T cell hybridoma cells (see antigen presentation assay). IL-2 produced by P32.2 cells was measured by ELISA in 48h culture supernatants. IL-10 production by B-IL-10 and B-MOG-IL-10 was measured by IL-10 ELISA in B cell supernatants 20h after B cell transduction.

### Antigen presentation assay

The ability of B-MOG and B-MOG-IL-10 cells to directly present MOG antigen was assessed with the MOG T cell hybridoma P32.2 (16506290). B cells were purified and transduced as described above, and co-cultured with 1x105 P32.2 cells at varying numbers of B cells in 200µl in flat bottom 96 well plate (Greiner) in complete RPMI medium for 48 hours. After this time, supernatants were collected and IL-2 ELISA was performed. P32.2 MOG-specific T cells hybridoma was maintained in complete RPMI medium at an optimal cell density of 1x10⁵ cells/ml in 24 well cell-culture plates (Greiner).

### Preparation of CNS-infiltrating mononuclear cells

Mice were sacrificed by CO₂ asphyxiation and perfused with cold PBS. Brains were cut into small pieces and digested with 10 mg/ml collagenase (Worthington Biochemical) and 1 mg/ml deoxyribonuclease (Sigma-Aldrich) for 30 min at 37°C followed by mechanical disaggregation. Single cell suspensions were washed once in RPMI culture medium. Mononuclear cells were prepared from the interface of a 30:70% discontinuous Percoll gradient after centrifugation for 20 min at 2000 rpm.

### Antibodies and Flow-cytometric analysis

Spleens and lymph nodes from EAE-induced mice and from naive mice were removed, and single cell suspensions were obtained by passing spleens through stainless steel meshes. Red blood cells were lysed with NH4CI lysis buffer. Mononuclear cells were obtained as described above. Cells (106) were incubated at 4°C with anti-murine FcgR (2.4G2) to block unspecific antibody binding. After 20 min, cells were stained with the following Abs: biotin-anti-CD19 (1D3), Cy5/Fitc anti-CD4 (GK 1.5), PercP-anti-CD8α (53-6.7), Cy5-anti-CD11b (M1/70), APC-anti-CD40L (MR1), Fitc-anti-MHCII (M5/114), PE-anti-CD45 (LCA Ly-5), biotin-anti-CD80 (16-10A1), biotin-anti-CD86 (GL-1), Cy5-anti-hCD4 (TT1), Fitc-anti-CD44 (IM7), biotin-anti-CD40 (3/23), biotin-anti CD69 (H1.2F3), PE/APC-anti-IFN-γ (XMG1.2), PE-anti-TNF-α (MP6-XT22), PE-anti-IL-17 (TC11-18H10), PE-anti-FoxP3 (FJK-16a) and APC anti-BrdU (B44). Antibodies were purchased from BD Pharmingen (San Diego, CA), eBioscience (San Diego, CA), Miltenyi Biotec (Bergisch Gladbach, Germany) or produced in our facility.

### In vitro restimulation and intracellular cytokine staining

Spleen, lymph nodes and brain infiltrating-mononuclear cells were diluted in RPMI medium supplemented with 10% FCS, L-glutamine, sodium pyruvate, penicillin, streptomycin and β-mercaptoethanol, and 4 x 106 cells were cultured in a volume of 1ml at 37°C with or without the addition of MOG(35-55) (10µg/ml) or with anti-CD3 (145-2C11) and anti CD28 (7D4) antibodies at 0.1µg/m an 1µg/ml respectively together with GolgiStop (Cytofix/Cytoperm kit; BD Pharmingen). After 5h 30min of incubation, cells were washed with FACS buffer (PBS, 0.2% (w/v) BSA, 0.02% (w/v) NaN3) and incubated with anti-mAbs for 20 min on ice and washed twice with FACS buffer. When biotinylated mAbs were used, another step of incubation with FITC streptavidin, APC streptavidin or PerCP streptavidin were performed under the same conditions s described above. For intracellular cytokine staining, cells were fixed whith fixation buffer (Cytofix/Cytoperm, BD bioscience) for 20 min. on ice, washed twice in permeabilization buffer (PermWash, BD bioscience) and resuspended in 50µl of permeabilization buffer containing the anti-cytokine Abs and incubated on at 4°C in the dark for 30 min. Next, cells were washed with FACS buffer and analysed FACS data were collected on a FACSCalibur (BD Biosciences), and data were analyzed using FlowJo software (Tree Star Inc.).

### Foxp3 and BrdU staining

Mice were treated with 1 mg BrdU (BD bioscience) in PBS i.p. 12 hours before analysis. Following harvest, BrdU incorporation was detected using an APC-BrdU Flow Kit (BD Pharmingen) largely as described in the manufacturer's instructions. To costain optimally for BrdU and Foxp3, it was essential to incorporate the use of the eBioscience fix/perm buffer (from the Foxp3 staining kit) as previously described (17617587).

### Preparation of spinal cord and histology.

Spines were removed from mice by cutting at the cervical and at the lumbar part. The flesh around the spine was removed and the spine was submerged in Formalin 10% and kept for a maximum of 48 hours at RT and embedded in paraffine until sections of the spine were cut.

For immunostaining, 2-3µm thick sections of formalin-fixed, paraffin-embedded tissue was cut, deparaffinized, and subjected to a heat induced epitope retrieval step. Slides were rinsed in cool running water, washed in Tris-buffered saline (pH 7.4) before incubation with primary polyclonal rabbit antibodies against CD3 (#N1580, Dako, Glostrup, Denmark, dilution 1:10) or Iba-1 (#019-19741, Wako, Richmond, VA, USA, 1:10000) for 30 minutes. For detection biotinylated donkey anti-rabbit (Dianova, Hamburg, Germany) secondary antibody was used followed by the streptavidinAP kit (K5005, Dako). Alkaline phosphatase was revealed by Fast Red as chromogen.

Luxol Fast Blue/ Periodic Acid Schiff (LFB/PAS) for the staining of the myelin sheath was performed on spinal cord slides.Slides were submerged in LFB 1 % (Sigma Aldrich) for 3h at 60°C. They were then rinsed in 95% ethanol, and postfixed in Lithium Carbonate (Roth) 0,05% for 5 sec., rinsed in 100% ethanol, in water and finally washed in water. For PAS staining were submerged in periodic acid solution for 5 min,rinsed in water and submerged in Schiff reagent for 15 min. The sections were finally washed with water and dehydrated.

### Generation of bone marrow-derived macrophages and suppression assay

For generation of bone marrow (BM)-derived macrophages, BM cells were platted overnight in a cell culture petri dish. Therefore, differentiated cells adhere more rapidly and precursors can be removed into a bacteria petri dish and be resuspended in the 10 ml RPMI medium containing 10% FCS/1% Pen-Strep, 2-ME, L-glutamine and 10% of growth factor M-CSF (produced in supernatant of X-63 cell line). At day 3 and day 6 of culture, cells were washed in order to remove non-adherent cells and new medium was replaced. On day 7, cells were harvested, counted and cultured in 96-well plated at 1.25x10⁵ cells per well in 50µl of RPMI. BM-macrophages were incubated with 100 µl of B-cell supernantants, medium containing 20ng/µl of recombinant mouse IL-10 (Sigma-Aldrich) or medium were added to the culture for 30 min. After this time, 50µl of RPMI medium containing 1µ/ml of CpG (ODN 1826, Invivogen) were added to the culture. TNF-a was measured in culture supernatants after 48 using a mosue TNF-a-ELISA kit (eBioscience).

### DESCRIPTION OF THE FIGURES

- Figure 1.: Effective transduction of resting mouse B cells with a HIV-based lentiviral vector
- Figure 2:: Engineering of IL-10-secreting antigen-presenting quiescent B cells
- Figure 3.: Engineered resting B cells provide protection from chronic EAE
- Figure 4.: Engineered resting B cells suppress T and B cell responses in secondary lymphoid organs
- Figure 5.: Engineered resting B cells suppress inflammatory T cell responses of T_{H}1 and T_{H}17 types in spleen
- Figure 6.: Engineered resting B cells protect recipient mice from CNS infiltration and demyelination
- Figure 7.: Engineered resting B cells suppress accumulation of inflammatory T cells in CNS
- Figure 8.: Engineered resting B cells do not affect natural Treg during EAE
- Figure 9.: Treatment with pDBR-MOG-tranduced MHC-II-deficient B cells

### DETAILED DESCRIPTION OF THE FIGURES

**Figure 1****. Effective transduction of resting mouse B cells with a HIV-based lentiviral vector. (a)** shows a schematic representation of the pDBR vector. Isolated B cells were transduced with pDBR, or left untransduced, and analyzed 18 h later for surface expression of hCD4. Histogram plot shows intensity of hCD4 surface expression on transduced B cells (plain line) and untransduced B cells (dotted line). (**b)** B cells were left untransduced (untr. B cells; dotted line), transduced with pDBR-hCD4 (B-hCD4 cells; black line), or left untransduced and activated with LPS (LPS; grey line). Cells were analyzed by flow cytometry for surface expression levels of MHC-II, CD80, CD86, CD40, CD69, and CD44 after 18h. Analyses were done after gating on live CD19⁺PI⁻ cells, except for B-hCD4 that were gated on CD19⁺PI⁻hCD4⁺. Histograms show representative stainings. (**c)** shows mean fluorescence intensity (MFI) for the stainings described in (b), compiling the data of 3 independent experiments, (**d)** shows IL-6 amounts in culture supernatants from the three types of B cells at 18h post-transduction for 4 independent experiments. (**e**) B cells transduced with pDBR were injected intravenously into C57BL/6 mice. Recipient mice were analyzed 18 h later to determine expression levels of the activation markers on B-hCD4 cells present in spleen. It is demonstrated that the preferred B cells really remain quiescent (nearly identical to the untreated control). Data are compiled from two independent experiments). Results are expressed as mean±s.e.m.;*, P<0.05. **, P<0.01. ***, P<0.0001.

**Figure 2****: Engineering of IL-10-secreting antigen-presenting quiescent B cells. (a)** B cells were transduced with pDBR-hCD4 (B-hCD4 cells), or pDBR-MOG and pDBR-IL-10 (B-MOG-IL-10 cells), or pDBR-IL-10 (B-IL-10 cells), and IL-10 was quantified in culture supernatants at 24 hours post-transduction (n=8).**(b)** B cells were transduced as described in (a) and their culture supernatants were collected at 18h post-transduction. These supernatants were then added to CpG-activated macrophages to measure their effect on TNF-α secretion. Recombinant mouse IL-10 (rmlL-10) was used as positive control. (n=4). **(c)** The MOG-reactive T cell hybridoma P32.2 (1 10⁵ cells) was stimulated with 2-fold increasing numbers of untransduced B cells (white squares), untransduced B cells pulsed with MOG(35-55) (black squares), B-MOG cells (white circles), B-MOG-IL-10 cells (white diamonds), or B-mock cells (black triangles). IL-2 production was measured after 48h (n=4). **(d)** P32.2 cells were cultured as in c with untransduced B cells (white diamonds), untransduced B cells pulsed with MOG(35-55) (black squares), B-MOG cells (white circles), or both B-MOG cells and an anti-MHC-II blocking antibody (grey triangles). IL-2 production was measured after 48h (n=4). Results are expressed as mean±s.e.m. Each experiment was repeated at least 3 times.

**Figure 3****. Engineered resting B cells provide protection from chronic EAE. (a)** Schematic representation of the B cell-based therapeutic protocol. **(b)** Mice were treated intravenously with 1 10⁷ B-MOG-IL-10 cells (white squares), or B-mock cells (grey squares) on days -6 and -2 before EAE induction or not treated (black squares). Mice were then assessed daily for EAE disease scores. Data show mean EAE scores ±s.e.m. (**c)** Mice were treated with B-MOG-IL-10 cells (white squares), or B-MOG cells (white diamonds), or B-IL-10 cells (grey diamonds) on days -6 and -2 before EAE induction or not treated (black squares), (**d)** Mice were treated with IL-10-deficient B cells transduced with pDBR-MOG (B_{IL-10-/-}-MOG) (white triangles), or B-MOG cells (white diamonds) on days -6 and -2 before EAE induction, or not treated (black squares). (**e)** Mice received an anti-CD25 antibody (PC61) intravenously to inactivate natural CD4⁺CD25⁺ T regulatory cells on day -3. Mice were additionally treated with B-MOG cells (white circles), or B-mock cells (grey circles) on days -6 and -2 before EAE induction, or not treated (black circles). Each experiment comprised 5 mice per group, and was repeated at least 3 times.

**Figure 4****. Engineered resting B cells suppress T and B cell responses in secondary lymphoid organs.** Mice were treated on days -6 and -2 with B-MOG cells (white squares), or B-mock cells (grey squares), or untreated (black squares), and analyzed at different time points after EAE induction Cells were isolated from spleens and draining lymph nodes, and either re-stimulated with MOG(35-55) for 6 hours *ex vivo*, or left unstimulated as controls. (**a)** shows representative stainings for surface CD4 and intracellular CD40L for splenocytes from mice at day 10 after EAE induction. Numbers indicate frequencies of CD40L⁺ among CD4⁺ T cells. (**b, c)** Numbers of CD4⁺CD40L⁺ T cells calculated from these frequencies in draining lymph nodes (**b**) and spleen (**c**) of mice analyzed at indicated time points. (**d**) Relative titers of MOG(35-55)-reactive IgM and IgG in serum at day 21 after EAE induction were determined by ELISA. This indicates lower antigen-specific antibody titers upon pre-treatment with B cells according to a preferred embodiment of the invention and then adding an antigen, compared to mock-treated or untreated control. This is especially indicative for the use of the B cells to prevent immune reactions against protein therapeutics. Each experiment comprised 4-5 mice per group, and was repeated 3 times. Results are expressed as mean±s.e.m.

**Figure 5****. Engineered resting B cells suppress inflammatory T cell responses of T_{H}1 and T_{H}17 types in spleen.** Splenocytes from mice treated on days -6 and -2 with B-MOG cells (white squares), or B-mock cells (grey squares), or from untreated mice (black squares) were taken at different time points after EAE induction, and re-stimulated with MOG(35-55) for 6 hours to measure production of cytokines by CD4⁺ and CD8⁺ T cells by flow cytometry. (**a)** shows representative intra-cellular stainings for IFN-γ, IL-17, and TNF-α in CD4⁺T cells taken from mice at day 10 after EAE induction and re-stimulated for 6h with MOG(35-55) *ex vivo.* Numbers indicate frequencies of cytokine-positive cells among CD4⁺ T cells. (**b, c, d, e)** Numbers of CD4⁺IFN-γ⁺ **(b)**, CD4⁺IL-17⁺ **(c),** CD4⁺TNF-α⁺ **(d),** and CD8⁺IFN-γ^{□□} **(e)** T cells per spleen calculated from these frequencies. Each experiment was repeated at least 2 times with 4-5 mice per group per time point. Results are expressed as mean±s.e.m.

**Figure 6****. Engineered resting B cells protect recipient mice from CNS infiltration and demyelination. (a)** Spinal cords from mice treated on days -6 and -2 with B-MOG cells, or B-mock cells, or from untreated mice were collected at day 21 after EAE induction. Sections were stained for myelination (luxol fast blue/ periodic-acid-Schiff), cell infiltration (hematoxylin and eosin), T cells (CD3), and macrophages (lba-1). Spinal cord sections from naive mice were used as controls. A representative picture from sections of a mouse per group is shown. (**b)** Spinal cords were prepared on day 35 after EAE induction from mice administered on day -3 with anti-CD25 antibody, and additionally treated on days -6 and -2 with either B-MOG cells, or B-mock cells. A group of mice (untreated) received the anti-CD25 antibody but no B cells. Scale bar: small quadrant=100µm, overview=500µm.

**Figure 7****. Engineered resting B cells suppress accumulation of inflammatory T cells in CNS.** Mononuclear cells were isolated from brains of mice treated on days -6 and -2 with B-MOG cells (white squares), or B-mock cells (grey squares), or from untreated mice (black squares) at indicated time points after EAE induction. Cell infiltrates were then characterized by flow cytometry. **(a)** Numbers of CD45^{high} cells per brain. **(b)** Numbers of CD11b⁺ myeloid cells per brain. **(c)** Numbers of CD4⁺ T cells per brain. (**d)** Numbers of CD8⁺ T cells per brain.**(e, f, g)** Cells were stimulated *ex-vivo* with anti-CD3+anti-CD28 antibodies for 6 hours, and cytokine production was quantified in CD4⁺ and CD8⁺ T cells by intra-cellular staining and flow cytometry. **(e)** Number of CD4⁺IL-17⁺ T cells per brain. **(f)** Number of CD4⁺IFN-γ⁺ T cells per brain. **(g)** Number of CD8⁺IFN-γ⁺T cells per brain. Each experiment comprised 4-5 mice per group per time point, and each analysis was repeated at least 2 times. Results are expressed as mean±s.e.m.

**Figure 8****. Engineered resting B cells do not affect natural Treg during EAE.** Mice were treated on days -6 and -2 with B-MOG cells (white squares), or B-mock cells (grey squares), or not treated mice (black squares), and analyzed at indicated time points after EAE induction. Natural regulatory T cells were characterized by staining for CD4 and Foxp3. **(a)** Number of CD4⁺Foxp3⁺ T regulatory cells per spleen or brain. **(b)** Frequency of natural Foxp3⁺ T regulatory cells among CD4⁺ T cells in spleen or brain. **(c, d)** Mice were administered with 1 mg BrdU 12h before analysis to characterize proliferative activity of regulatory T cells. **(c)** shows representative stainings gated on CD4⁺ T cells for Foxp3 and BrdU. **(d)** Frequency of proliferating BrdU⁺ cells among CD4⁺Foxp3⁺ T regulatory cells in spleen or brain. Each experiment comprised 4-5 mice per group per time point, and was repeated at least 2 times. Results are expressed as mean±s.e.m.

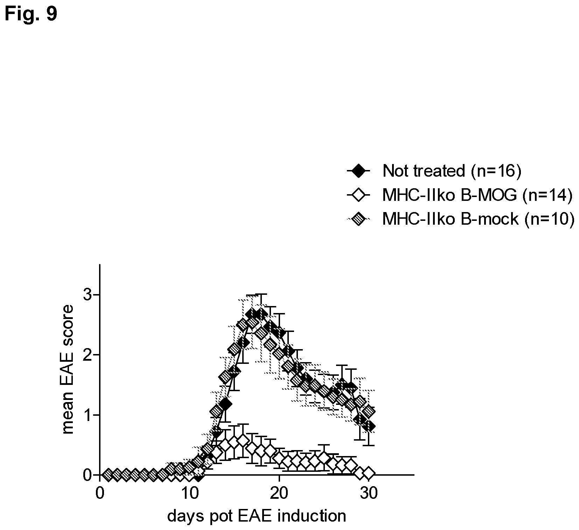

**Figure 9****. Treatment with pDBR-MOG-tranduced MHC-II-deficient B cells.** C57BL/6 mice received between 8 and 10 x 10⁶ transduced MHC-IIko-B cells in 200µl of PBS i.v. on days -6 and -2 before EAE induction. One group of mice received MHC-II knock-out (ko) B cells transduced with pDBR-MOG (MHC-IIko-B-MOG) and another group of mice received MHC-IIko B cells transduced with empty pDBR (MHC-IIko B-mock). A third group of mice did not receive B cells. On day 0, EAE was induced by immunization with 50µg MOG (35-55) in CFA. 240ng of Pertussis Toxin per mouse was injected on day 0, and day +2. Mice were monitored for signs of disease. The graph represents the compilation of 3 separate experiments.

## Claims

1. Modified naive B cells, **characterised in that**
- at least one antigen and/or epitopes thereof are presented on the cell surface, whereby
- the presented antigen(s) is encoded by and expressed from a lentiviral vector within said modified naïve B cell,
wherein said cells exhibit immune supressing properties against an antigen-specific immune reaction directed against the antigen encoded by said vector.

2. Modified B cells according to claim 1, **characterised in that** the modified B cells express similar levels of MHC-II (HLA), CD40, CD44, CD69, CD80, and/or CD86 as endogenous naive B cells, and preferably do not produce IL-6.

3. Modified B cells according to any one of the preceding claims, **characterised in that** the antigen of interest and/or epitopes thereof are presented via an antigen-presenting molecule on the surface of the modified B cells.

4. Modified B cells according to any one of the preceding claims, **characterised in that** the B cells secrete immunosuppressive molecules at levels higher than endogenous naive B cells.

5. Modified B cells according to the preceding claim, **characterised in that** the immunosuppressive molecule is IL-10.

6. Modified B cells according to any one of the preceding claims, in which an antigen-presenting molecule and/or a co-stimulatory molecule involved in presentation of antigen is additionally modulated.

7. Modified B cells according to the preceding claim, **characterised in that** the antigen-presenting molecule or co-stimulatory molecule involved in presentation of antigen, preferably MHC-II (HLA), is inhibited, blocked, suppressed, activated or over-expressed.

8. Method for producing modified antigen-presenting naive B cells according to any one of claims 1 to 7, **characterised by** providing isolated naive B cells, preferably by isolation of CD19+ cells, followed by transformation of the isolated B cells by transduction of a lentiviral vector, which encodes the antigen to be presented.

9. Use of a lentiviral vector pDBR according to SEQ ID NO 1 or a derivative thereof, which encodes an antigen to be presented, for the transformation of naive B cells and the production of modified antigen-presenting naive B cells according to claims 1 to 7.

10. Modified B cells according to any one of claims 1 to 7 for use as a medicament for the induction of immune tolerance and/or the suppression and/or inhibition of immune reactions, preferably antigen-specific immune reactions directed against the antigen encoded by the exogenous nucleic acid and/or unwanted adaptive immune reactions mediated by CD4+ T cells, CD8+ T cells, B cells and/or antibodies.

11. Modified B cells for use as a medicament according to claim 10, **characterised in that** the suppression of immune reaction is associated with the inhibition and/or prevention of an inflammatory T cell response, preferably by suppression of IFN-γ-, IL-17- and/or TNF-α-producing CD4+ or CD8+ T cells, especially of TH1 and/or TH17 cell types.

12. Modified B cells for use as a medicament according to any one of claims 10 or 11, **characterised in that** self-reactive humoral immune responses, such as autoimmune reactions, are suppressed, preferably by reducing the levels of antibodies directed against the antigen encoded by the exogenous nucleic acid

13. Modified B cells for use as a medicament according to any one of claims 10 to 12, **characterised in that**
- antigen-specific immune reactions, which are directed against an antigen expressed in the course of gene therapy, are suppressed, or
- immune reactions against therapeutic proteins and peptides, such as therapeutical antibodies, cytokines, enzymes or any other protein administered to a patient, are suppressed.

14. Pharmaceutical composition comprising modified B cells according to any one of claims 1 to 7 and at least one pharmaceutically acceptable carrier.

15. Modified B cells according to any one of claims 1 to 7 for use as a medicament in the treatment of unwanted immune reactions, such as those involved in autoimmune disorders, immune reactions to therapeutic proteins, and/or allergies.

## Patentansprüche

1. Modifizierte inaktive B-Zellen, **dadurch gekennzeichnet, dass**
- mindestens ein Antigen und/oder Epitope davon auf der Zelloberfläche präsentiert werden, wobei
- das/die präsentierte(n) Antigen(e) durch einen lentiviralen Vektor innerhalb der modifizierten inaktiven B-Zelle codiert und von diesem exprimiert wird,
wobei die Zellen immununterdrückende Eigenschaften gegen eine antigenspezifische Immunreaktion zeigen, die gegen das durch den Vektor codierte Antigen gerichtet ist.

2. Modifizierte B-Zellen nach Anspruch 1, **dadurch gekennzeichnet, dass** die modifizierten B-Zellen ähnliche Niveaus von MHC-II (HLA), CD40, CD44, CD69, CD80 und/oder CD86 wie endogene inaktive B-Zellen exprimieren und vorzugsweise kein IL-6 erzeugen.

3. Modifizierte B-Zellen nach einem der vorhergehenden Ansprüche, **dadurch gekennzeichnet, dass** das Antigen von Interesse und/oder Epitope davon über ein Antigen-präsentierendes Molekül auf der Oberfläche der modifizierten B-Zellen präsentiert wird/werden.

4. Modifizierte B-Zellen nach einem der vorhergehenden Ansprüche, **dadurch gekennzeichnet, dass** die B-Zellen immununterdrückende Moleküle in höheren Niveaus als endogene inaktive B-Zellen sezernieren.

5. Modifizierte B-Zellen nach dem vorhergehenden Anspruch, **dadurch gekennzeichnet, dass** das immununterdrückende Molekül IL-10 ist.

6. Modifizierte B-Zellen nach einem der vorhergehenden Ansprüche, bei denen ein Antigen-präsentierendes Molekül und/oder ein co-stimulierendes Molekül, das an der Antigenpräsentation beteiligt ist, zusätzlich moduliert ist.

7. Modifizierte B-Zellen nach dem vorhergehenden Anspruch, **dadurch gekennzeichnet, dass** das Antigen-präsentierende Molekül oder das co-stimulierendes Molekül, das an der Antigenpräsentation beteiligt ist, vorzugsweise MHC-II (HLA), gehemmt, blockiert, unterdrückt, aktiviert oder überexprimiert ist.

8. Herstellungsverfahren von inaktiven B-Zellen mit Antigenen nach einem der Ansprüche 1 bis 7, **gekennzeichnet durch** Bereitstellen isolierter inaktiver B-Zellen, vorzugsweise durch Isolierung von CD19+-Zellen, gefolgt von einer Transformation der isolierten B-Zellen durch Transduktion eines lentiviralen Vektors, der für das zu präsentierende Antigen codiert.

9. Verwendung eines lentiviralen Vektors pDBR gemäß SEQ ID NO 1 oder eines Derivats davon, welcher/welches für ein zu präsentierendes Antigen codiert, für die Transformation von inaktiven B-Zellen und eine Herstellung von inaktiven B-Zellen mit Antigenen gemäß den Ansprüchen 1 bis 7.

10. Modifizierte B-Zellen nach einem der Ansprüche 1 bis 7 zur Verwendung als Medikament für die Induktion einer Immuntoleranz und/oder Unterdrückung und/oder Hemmung von Immunreaktionen, vorzugsweise antigenspezifischen Immunreaktionen, die gegen das durch die exogenen Nukleinsäure codierte Antigen gerichtet sind, und/oder unerwünschte adaptive Immunreaktionen, die durch CD4+-T-Zellen, CD8+-T-Zellen, B-Zellen und/oder Antikörper vermittelt werden.

11. Modifizierte B-Zellen zur Verwendung als Medikament nach Anspruch 10, **dadurch gekennzeichnet, dass** die Unterdrückung einer Immunreaktion mit der Hemmung und/oder Verhinderung einer inflammatorischen T-Zellreaktion verbunden ist, vorzugsweise durch Unterdrückung von IFN-γ-, IL-17- und/oder TNF-α-herstellenden CD4+- oder CD8+-T-Zellen, insbesondere von TH1- und/oder TH17-Zelltypen.

12. Modifizierte B-Zellen zur Verwendung als Medikament nach einem der Ansprüche 10 oder 11, **dadurch gekennzeichnet, dass** selbstreaktive humorale Immunreaktionen, wie Autoimmunreaktionen, unterdrückt werden, vorzugsweise durch Reduzieren der Spiegel von Antikörpern, die gegen das durch die exogene Nukleinsäure codierte Antigen gerichtet sind.

13. Modifizierte B-Zellen zur Verwendung als Medikament nach einem der Ansprüche 10 bis 12, **dadurch gekennzeichnet, dass**
- antigenspezifische Immunreaktionen, die gegen ein im Verlauf einer Gentherapie exprimiertes Antigen gerichtet sind, unterdrückt werden oder
- Immunreaktionen gegen therapeutische Proteine und Peptide, wie therapeutische Antikörper, Zytokine, Enzyme oder ein beliebiges anderes Protein, die/das einem Patienten verabreicht werden/wird, unterdrückt werden.

14. Pharmazeutische Zusammensetzung, umfassend modifizierte B-Zellen nach einem der Ansprüche 1 bis 7 und mindestens einen pharmazeutisch annehmbaren Träger.

15. Modifizierte B-Zellen nach einem der Ansprüche 1 bis 7 zur Verwendung als Medikament bei der Behandlung unerwünschter Immunreaktionen, wie solchen, die an Autoimmunerkrankungen, Immunreaktionen auf therapeutische Proteine und/oder Allergien beteiligt sind.

## Revendications

1. Lymphocytes B naïfs modifiés, **caractérisés en ce que**
- au moins un antigène et/ou épitope de ceux-ci sont présentés sur la surface cellulaire,
- le ou les antigènes présentés étant codés et exprimés par un vecteur lentiviral présent dans ledit lymphocyte B naïf modifié,
lesdites cellules présentant des propriétés de suppression immunitaire contre une réaction immunitaire spécifique d'un antigène dirigée contre l'antigène codé par ledit vecteur.

2. Lymphocytes B modifiés selon la revendication 1, **caractérisés en ce qu'**ils expriment CMH-II (HLA), CD40, CD44, CD69, CD80 et/ou CD86 à des teneurs similaires à celles exprimées par les lymphocytes B naïfs endogènes, et, de préférence, ne produisent pas d'IL-6.

3. Lymphocytes B modifiés selon l'une quelconque des revendications précédentes, **caractérisés en ce que** l'antigène d'intérêt et/ou ses épitopes sont présentés par l'intermédiaire d'une molécule présentant l'antigène sur la surface des lymphocytes B modifiés.

4. Lymphocytes B modifiés selon l'une quelconque des revendications précédentes, **caractérisés en ce qu'**ils sécrètent des molécules immunosuppressives à des teneurs plus élevées que les lymphocytes B naïfs endogènes.

5. Lymphocytes B modifiés selon l'une quelconque des revendications précédentes, **caractérisés en ce que** la molécule immunosuppressive est l'IL-10.

6. Lymphocytes B modifiés selon l'une quelconque des revendications précédentes, dans lesquels une molécule présentant l'antigène et/ou une molécule co-stimulatrice impliquée dans la présentation de l'antigène est en outre modulée.

7. Lymphocytes B modifiés selon la revendication précédente, **caractérisés en ce que** la molécule présentant l'antigène et/ou la molécule co-stimulatrice impliquée dans la présentation de l'antigène, de préférence CMH-II (HLA), est inhibée, bloquée, supprimée, activée ou surexprimée.

8. Procédé de production de lymphocytes B naïfs présentant l'antigène modifiés selon l'une quelconque des revendications 1 à 7, **caractérisé par** l'obtention de lymphocytes B isolés, de préférence par isolement de cellules CD19+, suivie de la transformation des lymphocytes B isolés par transduction d'un vecteur lentiviral, qui code l'antigène à présenter.

9. Utilisation d'un vecteur lentiviral pDBR conforme à SEQ ID NO 1 ou à un dérivé de celle-ci, qui code un antigène à présenter, pour la transformation de lymphocytes B naïfs et la transformation de lymphocytes B naïfs et la production de lymphocytes B naïfs présentant l'antigène modifiés selon les revendications 1 à 7.

10. Lymphocytes B modifiés selon l'une quelconque des revendications 1 à 7, destinés à être utilisés comme médicament pour l'induction d'une tolérance immunitaire et/ou la suppression et/ou l'inhibition de réactions immunitaires, de préférence des réactions immunitaires spécifiques d'un antigène dirigées contre l'antigène codé par l'acide nucléique exogène et/ou des réactions immunitaires adaptatives non désirées médiées par des lymphocytes T CD4+, des lymphocytes T CD8+ et/ou des anticorps.

11. Lymphocytes B modifiés destinés à être utilisés comme médicament selon la revendication 10, **caractérisés en ce que** la suppression de la réaction immunitaire est associée à l'inhibition et/ou à la prévention de la réponse en lymphocytes T inflammatoire, de préférence la suppression de lymphocytes T CD4+ ou CD8+ produisant l'IFN-γ, l'IL-17 et/ou le TNF-α, en particulier les types cellulaires TH1 et/ou TH17.

12. Lymphocytes B modifiés destinés à être utilisés comme médicament selon l'une quelconque des revendications 10 ou 11, **caractérisés en ce que** les réponses immunitaires humorales auto-réactives, telles que les réactions auto-immunes, sont supprimées, de préférence par réduction des teneurs en anticorps dirigés contre l'antigène codé par l'acide nucléique exogène.

13. Lymphocytes B modifiés destinés à être utilisés comme médicament selon l'une quelconque des revendications 10 à 12, **caractérisés en ce que**
- les réactions immunitaires spécifiques d'un antigène, qui sont dirigées contre un antigène exprimé au cours de la thérapie génique, sont supprimées, ou
- les réactions immunitaires contre des protéines et des peptides thérapeutiques, comme des anticorps, des cytokines et des enzymes thérapeutiques ou toute autre protéine administrée à un patient, sont supprimées.

14. Composition pharmaceutique comprenant des lymphocytes B modifiés selon l'une quelconque des revendications 1 à 7 et au moins un véhicule pharmaceutiquement acceptable.

15. Lymphocytes B modifiés selon l'une quelconque des revendications 1 à 7, destinés à être utilisés comme médicament dans le traitement de réactions immunitaires non désirées, comme celles impliquées dans les troubles auto-immuns, les réactions immunitaires contre des protéines thérapeutiques, et/ou les allergies.


### SEQUENCE LISTING

<110> Deutsches Rheuma-Forschungszentrum Berlin Fillatreau, Simon
<120> ANTIGEN-PRESENTING MODIFIED NAÏVE B CELLS FOR IMMUNE SUPPRESSION AND A METHOD FOR PRODUCING SAID MODIFIED CELLS
<130> XII 341/11
<150> EP 10075225.2
   <151> 2010-05-26
<160> 1
<170> PatentIn version 3.5
<210> 1
   <211> 6619
   <212> DNA
   <213> Artificial Sequence
<220>
   <223> Artificial sequence
<400> 1
   gtcgacggat cgggagatct cccgatcccc tatggtgcac tctcagtaca atctgctctg      60
   atgccgcata gttaagccag tatctgctcc ctgcttgtgt gttggaggtc gctgagtagt     120
   gcgcgagcaa aatttaagct acaacaaggc aaggcttgac cgacaattgc atgaagaatc     180
   tgcttagggt taggcgtttt gcgctgcttc gcgatgtacg ggccagatat acgcgttgac     240
   attgattatt gactagttat taatagtaat caattacggg gtcattagtt catagcccat     300
   atatggagtt ccgcgttaca taacttacgg taaatggccc gcctggctga ccgcccaacg     360
   acccccgccc attgacgtca ataatgacgt atgttcccat agtaacgcca atagggactt     420
   tccattgacg tcaatgggtg gagtatttac ggtaaactgc ccacttggca gtacatcaag     480
   tgtatcatat gccaagtacg ccccctattg acgtcaatga cggtaaatgg cccgcctggc     540
   attatgccca gtacatgacc ttatgggact ttcctacttg gcagtacatc tacgtattag     600
   tcatcgctat taccatggtg atgcggtttt ggcagtacat caatgggcgt ggatagcggt     660
   ttgactcacg gggatttcca agtctccacc ccattgacgt caatgggagt ttgttttggc     720
   accaaaatca acgggacttt ccaaaatgtc gtaacaactc cgccccattg acgcaaatgg     780
   gcggtaggcg tgtacggtgg gaggtctata taagcagcgc gttttgcctg tactgggtct     840
   ctctggttag accagatctg agcctgggag ctctctggct aactagggaa cccactgctt     900
   aagcctcaat aaagcttgcc ttgagtgctt caagtagtgt gtgcccgtct gttgtgtgac     960
   tctggtaact agagatccct cagacccttt tagtcagtgt ggaaaatctc tagcagtggc    1020
   gcccgaacag ggacttgaaa gcgaaaggga aaccagagga gctctctcga cgcaggactc    1080
   ggcttgctga agcgcgcacg gcaagaggcg aggggcggcg actggtgagt acgccaaaaa    1140
   ttttgactag cggaggctag aaggagagag atgggtgcga gagcgtcagt attaagcggg    1200
   ggagaattag atcgcgatgg gaaaaaattc ggttaaggcc agggggaaag aaaaaatata 1260
   aattaaaaca tatagtatgg gcaagcaggg agctagaacg attcgcagtt aatcctggcc 1320
   tgttagaaac atcagaaggc tgtagacaaa tactgggaca gctacaacca tcccttcaga 1380
   caggatcaga agaacttaga tcattatata atacagtagc aaccctctat tgtgtgcatc 1440
   aaaggataga gataaaagac accaaggaag ctttagacaa gatagaggaa gagcaaaaca 1500
   aaagtaagac caccgcacag caagcggccg gccgctgatc ttcagacctg gaggaggaga 1560
   tatgagggac aattggagaa gtgaattata taaatataaa gtagtaaaaa ttgaaccatt 1620
   aggagtagca cccaccaagg caaagagaag agtggtgcag agagaaaaaa gagcagtggg 1680
   aataggagct ttgttccttg ggttcttggg agcagcagga agcactatgg gcgcagcgtc 1740
   aatgacgctg acggtacagg ccagacaatt attgtctggt atagtgcagc agcagaacaa 1800
   tttgctgagg gctattgagg cgcaacagca tctgttgcaa ctcacagtct ggggcatcaa 1860
   gcagctccag gcaagaatcc tggctgtgga aagataccta aaggatcaac agctcctggg 1920
   gatttggggt tgctctggaa aactcatttg caccactgct gtgccttgga atgctagttg 1980
   gagtaataaa tctctggaac agatttggaa tcacacgacc tggatggagt gggacagaga 2040
   aattaacaat tacacaagct taatacactc cttaattgaa gaatcgcaaa accagcaaga 2100
   aaagaatgaa caagaattat tggaattaga taaatgggca agtttgtgga attggtttaa 2160
   cataacaaat tggctgtggt atataaaatt attcataatg atagtaggag gcttggtagg 2220
   tttaagaata gtttttgctg tactttctat agtgaataga gttaggcagg gatattcacc 2280
   attatcgttt cagacccacc tcccaacccc gaggggaccc gacaggcccg aaggaataga 2340
   agaagaaggt ggagagagag acagagacag atccattcga ttagtgaacg gatcggcact 2400
   gcgtgcgcca attctgcaga caaatggcag tattcatcca caattttaaa agaaaagggg 2460
   ggattggggg gtacagtgca ggggaaagaa tagtagacat aatagcaaca gacatacaaa 2520
   ctaaagaatt acaaaaacaa attacaaaaa ttcaaaattt tcgggtttat tacagggaca 2580
   gcagagatcc agtttggtta gtaccgggcc gctcttgtga cgcgttaacg gagattccag 2640
   caggtcgagg gacctaataa cttcgtatag catacattat acgaagttat attaagggtt 2700
   ccaagcttaa gcggccgcgt ggataaccgt attaccgcca tgcattagtt attaatagta 2760
   atcaattacg gggtcattag ttcatagccc atatatggag ttccgcgtta cataacttac 2820
   ggtaaatggc ccgcctggct gaccgcccaa cgacccccgc ccattgacgt caataatgac 2880
   gtatgttccc atagtaacgc caatagggac tttccattga cgtcaatggg tggagtattt 2940
   acggtaaact gcccacttgg cagtacatca agtgtatcat atgccaagta cgccccctat 3000
   tgacgtcaat gacggtaaat ggcccgcctg gcattatgcc cagtacatga ccttatggga 3060
   ctttcctact tggcagtaca tctacgtatt agtcatcgct attaccatgg tgatgcggtt 3120
   ttggcagtac atcaatgggc gtggatagcg gtttgactca cggggatttc caagtctcca 3180
   ccccattgac gtcaatggga gtttgttttg gcaccaaaat caacgggact ttccaaaatg 3240
   tcgtaacaac tccgccccat tgacgcaaat gggcggtagg cgtgtacggt gggaggtcta 3300
   tataagcaga gctggtttag tgaaccgtca gatccgctag ccgcggatcc gcgctagtct 3360
   agactaggtt taaacccgga attcgccccg ctcgagcgga aattcgtcga gggacctaat 3420
   aacttcgtat agcatacatt atacgaagtt atacatgttt aagggttccg gttccactag 3480
   gtacaattcg atatcaagct tatcgataat caacctctgg attacaaaat ttgtgaaaga 3540
   ttgactggta ttcttaacta tgttgctcct tttacgctat gtggatacgc tgctttaatg 3600
   cctttgtatc atgctattgc ttcccgtatg gctttcattt tctcctcctt gtataaatcc 3660
   tggttgctgt ctctttatga ggagttgtgg cccgttgtca ggcaacgtgg cgtggtgtgc 3720
   actgtgtttg ctgacgcaac ccccactggt tggggcattg ccaccacctg tcagctcctt 3780
   tccgggactt tcgctttccc cctccctatt gccacggcgg aactcatcgc cgcctgcctt 3840
   gcccgctgct ggacaggggc tcggctgttg ggcactgaca attccgtggt gttgtcgggg 3900
   aaatcatcgt cctttccttg gctgctcgcc tgtgttgcca cctggattct gcgcgggacg 3960
   tccttctgct acgtcccttc ggccctcaat ccagcggacc ttccttcccg cggcctgctg 4020
   ccggctctgc ggcctcttcc gcgtcttcgc cttcgccctc agacgagtcg gatctccctt 4080
   tgggccgcct ccccgcatcg ataccgtcga cctcgatcga gacctagaaa aacatggagc 4140
   aatcacaagt agcaatacag cagctaccaa tgctgattgt gcctggctag aagcacaaga 4200
   ggaggaggag gtgggttttc cagtcacacc tcaggtacct ttaagaccaa tgacttacaa 4260
   ggcagctgta gatcttagcc actttttaaa agaaaagggg ggactggaag ggctaattca 4320
   ctcccaacga agacaagata tccttgatct gtggatctac cacacacaag gctacttccc 4380
   tgattggcag aactacacac cagggccagg gatcagatat ccactgacct ttggatggtg 4440
   ctacaagcta gtaccagttg agcaagagaa ggtagaagaa gccaatgaag gagagaacac 4500
   ccgcttgtta caccctgtga gcctgcatgg gatggatgac ccggagagag aagtattaga 4560
   gtggaggttt gacagccgcc tagcatttca tcacatggcc cgagagctgc atccggactg 4620
   tactgggtct ctctggttag accagatctg agcctgggag ctctctggct aactagggaa 4680
   cccactgctt aagcctcaat aaagcttgcc ttgagtgctt caagtagtgt gtgcccgtct 4740
   gttgtgtgac tctggtaact agagatccct cagacccttt tagtcagtgt ggaaaatctc 4800
   tagcagcatg tgagcaaaag gccagcaaaa ggccaggaac cgtaaaaagg ccgcgttgct 4860
   ggcgtttttc cataggctcc gcccccctga cgagcatcac aaaaatcgac gctcaagtca 4920
   gaggtggcga aacccgacag gactataaag ataccaggcg tttccccctg gaagctccct 4980
   cgtgcgctct cctgttccga ccctgccgct taccggatac ctgtccgcct ttctcccttc 5040
   gggaagcgtg gcgctttctc atagctcacg ctgtaggtat ctcagttcgg tgtaggtcgt 5100
   tcgctccaag ctgggctgtg tgcacgaacc ccccgttcag cccgaccgct gcgccttatc      5160
   cggtaactat cgtcttgagt ccaacccggt aagacacgac ttatcgccac tggcagcagc      5220
   cactggtaac aggattagca gagcgaggta tgtaggcggt gctacagagt tcttgaagtg      5280
   gtggcctaac tacggctaca ctagaagaac agtatttggt atctgcgctc tgctgaagcc      5340
   agttaccttc ggaaaaagag ttggtagctc ttgatccggc aaacaaacca ccgctggtag      5400
   cggtggtttt tttgtttgca agcagcagat tacgcgcaga aaaaaaggat ctcaagaaga      5460
   tcctttgatc ttttctacgg ggtctgacgc tcagtggaac gaaaactcac gttaagggat      5520
   tttggtcatg agattatcaa aaaggatctt cacctagatc cttttaaatt aaaaatgaag      5580
   ttttaaatca atctaaagta tatatgagta aacttggtct gacagttacc aatgcttaat      5640
   cagtgaggca cctatctcag cgatctgtct atttcgttca tccatagttg cctgactccc      5700
   cgtcgtgtag ataactacga tacgggaggg cttaccatct ggccccagtg ctgcaatgat      5760
   accgcgagac ccacgctcac cggctccaga tttatcagca ataaaccagc cagccggaag      5820
   ggccgagcgc agaagtggtc ctgcaacttt atccgcctcc atccagtcta ttaattgttg      5880
   ccgggaagct agagtaagta gttcgccagt taatagtttg cgcaacgttg ttgccattgc      5940
   tacaggcatc gtggtgtcac gctcgtcgtt tggtatggct tcattcagct ccggttccca      6000
   acgatcaagg cgagttacat gatcccccat gttgtgcaaa aaagcggtta gctccttcgg      6060
   tcctccgatc gttgtcagaa gtaagttggc cgcagtgtta tcactcatgg ttatggcagc      6120
   actgcataat tctcttactg tcatgccatc cgtaagatgc ttttctgtga ctggtgagta      6180
   ctcaaccaag tcattctgag aatagtgtat gcggcgaccg agttgctctt gcccggcgtc      6240
   aatacgggat aataccgcgc cacatagcag aactttaaaa gtgctcatca ttggaaaacg      6300
   ttcttcgggg cgaaaactct caaggatctt accgctgttg agatccagtt cgatgtaacc      6360
   cactcgtgca cccaactgat cttcagcatc ttttactttc accagcgttt ctgggtgagc      6420
   aaaaacagga aggcaaaatg ccgcaaaaaa gggaataagg gcgacacgga aatgttgaat      6480
   actcatactc ttcctttttc aatattattg aagcatttat cagggttatt gtctcatgag      6540
   cggatacata tttgaatgta tttagaaaaa taaacaaata ggggttccgc gcacatttcc      6600
   ccgaaaagtg ccacctgac                                                   6619